# EUROPEAN PATENT SPECIFICATION

(11) **EP 0 758 021 B1**
(45) Date of publication and mention of the grant of the patent: **29.03.2006**
(21) Application number: 96830445.1
(22) Date of filing: 02.08.1996
(51) Int. Cl.: C12Q 1/37, C07K 5/06, A61K 38/55

(54) **Method for determining the therapeutic activity of metalloproteinase inhibitor compounds, new inhibitor compounds, and the therapeutic use thereof**
Verfahren zur Bestimmung der therapeutischen Wirkung von Metalloproteinase-Verbindungen, neue Inhibitor-Verbindungen und deren therapeutische Verwendung
Méthode pour déterminer l'activité thérapeutique des inhibiteurs de métalloprotéinase, nouveaux composés inhibiteurs, et leur application thérapeutique

(30) Priority: 07.08.1995 IT RM950557
(43) Date of publication of application: 12.02.1997
(73) Proprietor: POLIFARMA S.p.A., 00155 Roma (RM) (IT)
(72) Inventor: Politi, Vincenzo, 00179 Rome (IT); D'Alessio, Silvana, 00174 Rome (IT); Di Stazio, Giovanni, 00136 Rome (IT); De Luca, Giovanna, 00136 Rome (IT); Materazzi, Mario, 00155 Rome (IT)
(74) Representative: Bazzichelli, Alfredo

(56) References cited:
- EP-A- 0 423 077
- EP-A- 0 489 579
- WO-A-91/05555
- WO-A-92/06108
- ROBEVA ET AL.: "Synthetic and endogeneous inhibitors of snakevenom metalloproteinases" BIOMEDICA BIOCHIMICA ACTA, vol. 50, no. 4-6, 1991, pages 769-773, XP000196518

## Description

### BACKGROUND OF THE INVENTION

### 1. FIELD OF THE INVENTION

The present invention relates to a method that makes it possible to determine, with a high level of accuracy, information on the therapeutic activity in mammals, including man, of a class of compounds of peptidomimetic nature that are inhibitors of metalloproteinase enzymes present in snake venom.

The invention further relates to new compounds of the class indicated above, as well as their therapeutic use in a large number of important human diseases, including tumoral invasion, rheumatoid arthritis, periodontitis, corneal ulcers, multiple sclerosis, aneurism of the aorta, osteoporosis, the cicatrization of wounds, contact dermatitis, arteriosclerosis, septic shock, parasite invasion, hypertension, allergies, defective immune response, Alzheimer's disease, chronic bronco-pulmonitis, pulmonary emphysema, cirrhosis of the liver, dilatational cardiomyopathy and disfunctions in the reproductive system.

### 2. DESCRIPTION OF THE PRIOR ART

Snake venoms are complex mixtures, containing a wide variety of proteolytic enzymes which have the job of digesting the prey or altering its physiological functions, especially as regards the cardiocirculatory system. In fact, it is well known (see for example Critical Reviews in Toxicology 21, 171-182, 1991) that the venom from snakes belonging to the family of the Viperidae have profound effects on the hemostatic and fibrinolytic systems, showing pro-coagulant or, alternatively, anti-coagulant activities. In a similar manner, factors that have a powerful inhibiting effect on blood platelet aggregation have been found, along with others that interfere with activation of prothrombin or fibrin.

An extremely important class of enzymes found in the venoms of snakes belonging to the family Crotalidae are the so-called Hemorrhagic Factors, or Hemorrhagins. These are structurally of use to the snake, as they rapidly induce extensive internal hemorrhages in the victims, causing circulatory collapse and preventing the victim from escaping its fate. The mechanism of the hemorrhagic action is due to the particular ease with which the enzymes are capable of degrading a large number of filiform proteins which hold bound between them the various vasal endothelial cells, allowing the elements of the blood to escape from the vessels. Recent studies (see for example Pharmacology and Therapeutics 62, 325-372, 1994) have made it possible to ascertain that the Hemorrhagic Factors, comprising a large number of enzymes isolated from venoms, have an extremely varied molecular weight (usually between 20 and 90 KDa), and often contain a number of functional sub-units, delegated for hemorrhagic, blood platelet anti-aggregant and adhesive activities. Although their molecular weights differ greatly, the Hemorrhagins have several fixed characteristics on the catalytic site, in the way that Zinc bonds to certain amino acids in the proteic chain, and in the way in which they attack the proteins in the basal membrane of blood vessels.

The presence of Zinc in the active site is not exclusive to Hemorrhagins, but is characteristic of a wide number of proteolytic enzymes that perform important physiological and pathological functions in the organism of animals, from the smallest and most simple to the most highly evolved. Through the study of the sequences in residues from the proteic chain and the amino acids involved in Zinc bonding it has been possible to obtain a sort of "family tree" for this family of proteases (see for example FEBS Letters 312, 110-114, 1992): it has thus been seen that enzymes belonging to widely differing living beings, such as Astacin (extracted from a river crustacean), Serratin (obtained from a microorganism), Matrixins (present in the organism of mammals, where they have important effects on cell migration and the reconstruction of damaged tissue) and the Hemorrhage Factors of snake venoms, in reality differ only in one of the four amino acids binding Zinc in the active site, and can thus in a certain sense be considered as distantly related to each other. However, this does not mean that the functions performed by these enzymes are similar: it has, in fact, been clarified that the proteolytic enzymes of snake venoms have no similarity, either structural or functional with any other protein in the plant or animal world, with the exception of the Zinc site, whereas they are very similar to each other, and they all appear to derive from a single ancestral gene, so much so that it is possible to define a new family of proteinases: the snake venom metalloproteinases (see for example Biol.Chem. Hoppe-Seyler 373, 381-385, 1992). EP-A-0 489 579 describes peptidyl derivatives which are metalloprotease inhibitors and have a selective gelatinase action and may be used in the treatment of cancer to control the development of tumour metastasises.
WO-91/05555 describes inhibitors of the Zn-metalloendopeptidases, enkephalinase, angiotensin-converting enzyme and collagenase. The inhibitors peptide or peptide ester derivatives of N-acyl derivatives of amino acids.

### SUMMARY OF THE INVENTION

In the program of research that has resulted in the present invention, compounds were initially synthesized that have a powerful inhibitory activity on snake venom metalloproteinases: this was done with the evident intention of finding substances that are potentially of use to antagonize the toxic and lethal effects in persons suffering from snake bites, but also to evaluate the possible new pharmacological activities deriving from the structural similarities in the active site of venom Hemorrhagic Factors and other Zinc-dependent metalloproteinases (among which the Matrixins), present in the cells of mammals.

The starting point of the research program was provided by the long-term experience of the Applicant in the presence and function of numerous components found in snake venoms, and by the evidence that, as published in (Biomed. Biochim.Acta 50, 769-773, 1991), snakes protect themselves from the toxic effects of their own metalloproteinases by the production of two tripeptides, which act as competitive inhibitors on the enzyme. The first step was that of carrying out the synthesis of a new family of compounds of peptido-mimetic nature, having the feature that the initial tripeptide was replaced by chemical groups capable of improving affinity with the active site of the enzyme. A Hemorrhagin was then selected that is particularly sensitive to the proteolytic test used "in vitro", purified from the venom of Crotalus Adamanteus: in this way a satisfactory model was obtained on which to test the strength of the synthesized compounds. Finally, several of the new peptido-mimetic compounds were pharmacologically tested in models capable of foreseeing a possible therapeutic use of the new substances.

The results obtained, which form the basis of the present invention, are totally innovative, and enable it to be stated that, through synthesis of inhibitors of the enzymes produced by snake venom with an hemorrhagic function, it, is possible to develop a new method to antagonize the lethal effects of certain classes of snake venom, and a new system to obtain highly predictive information on important therapeutic activities in man, in a wide range of diseases in which the pathogenic intervention of Zinc-dependent metalloproteinases has been demonstrated, which range from tumoral invasion, to rheumatoid arthritis, to periodontitis, corneal ulcers, multiple sclerosis, aneurism of the aorta, osteoporosis, cicatrization of wounds, contact dermatitis, arteriosclerosis, septic shock, parasite invasion, hypertension, allergies, defective immune responses, Alzheimer's disease, chronic bronco-pulmonitis, pulmonary emphysema, cirrhosis of the liver, dilatational cardiomyopathy and malfunctions of the reproductive system.

An object of the present invention is therefore a method for determining the therapeutic activity in mammals of a compound of peptido-mimetic tipe to recognize and produce an active drug for human and animal treatment, comprising the steps of:
determining by a first enzyme inhibition test a first level of inhibitory activity of said compound as an inhibitor of zinc-dependent metalloproteinases extracted from the venom of snakes belonging to the families Crotalidae and Viperidae;
verifying said first level with respect to a threshold level of activity sufficient to define said compound as an inhibitor of said zinc-dependent metalloproteinases from snake venom;
determining by a second enzyme inhibition test a second level of inhibitory activity of said compound as an ihibitor of a zinc-dependent metalloproteinase indogenous in mammal organism and inducing pathological situations in said mammals;
verifying said second level with respect to a threshold level of activity sufficient to define said compound as an inhibitor of said zinc-dependent metalloproteinase endogenous in mammals; and
verifying by standard pharmacological tests the activity of said compound with respect to said pathological situation.

The present invention also has as an object compounds that can be used for human therapy in a large number of pathological situations, which range from snake bite poisoning to the invasion by tumoral cells, to rheumatoid arthritis and other forms of inflammation, to multiple sclerosis, to aneurysms of the aorta, to osteoporosis, to arteriosclerosis, to septic shock, to Alzheimer's disease, to allergies and so on; in any case, that is to say, in which the predominant pathological agent is a zinc-dependent metalloproteinase, whether produced by snake venom or synthesized within the cells of mammals.

### DESCRIPTION OF THE PREFERRED EMBODIMENTS

A characteristic common to the compounds described in the present invention is that of being good inhibitors of zinc-dependent metalloproteinases produced in large numbers by the snakes belonging to the species of vipers and rattlesnakes, which are one of the most important lethal factors resulting from the bites of these snakes.

As is known from a work published by the researchers authors of the present invention (Biomedica Biochimica Acta 50, 769-773, 1991), snake venoms produce large amounts of small peptides, which probably have the job of inhibiting metalloproteinases, so that these do not damage the tissues of the snake itself. However, these inhibitors have an extremely bland activity, because the enzyme must be freed when it is injected into the victim. Our aim was therefore that of achieving the synthesis of compounds in some way similar to the peptides found in the venoms, but with a much higher inhibiting activity (approximately 1000 time, in the best cases), and capable of being administered orally, as is the case with normal drugs.

It has now surprisingly been found, and forms the basis of the new method according to the present invention, that there is a close correspondence between inhibition of the enzymes found in snake venom and the pharmacological results obtained in animal models in which the pathological agent was presumed to be a zinc-dependent metalloproteinase produced by the tissues of the mammal, so that it is possible to conclude that snake venom zinc-dependent metalloproteinases form an excellent model for primary screening, and that inhibitors of said enzymes are potentially usable in all pathological situations induced by zinc-dependent metalloproteinases present in the organism of mammals.

As zinc-dependent metalloproteinases suitable to be used in the method according to the present invention the following are particularly indicated: Crotalus Adamanteus hemorrhagin, Crotalus Atrox hemorrhagin, Agkistrodon Bilineatus botropasin and hemorrhagin and a number of Echis Carinatus hemorrhagins extracted from the venom of the respective snakes.

The operation used to determine the level of inhibitory activity of the inhibitor compound on the metalloproteinase enzyme can be a test determining the amount of inhibitor required (IC50) to inhibit the enzyme's activity.

A numeric evaluation of the inhibitor activity is thus obtained, allowing selection based on activity level.

As mentioned above, it has been surprisingly found that this high level of inhibitory activity is an extremely reliable indication of the inhibitory activity of the compound in question even in the face of other metalloproteinases of endogenous nature in mammals, the activity of which is responsible for a wide range of disturbances and diseases in man.

The method according to the present invention therefore provides an instrument for the production of new drugs with direct and non-casual selection criteria, resulting in a reduction in costs.

On the basis of the method according to the invention a number of new inhibitor compounds having therapeutic activity have also been discovered.

Additional compounds described in the present invention are not novel, but are the object of the prior US patent No. 5,504.071 corresponding to PCT WO92/06108 to the same Applicant. Said known compounds all belong to the class of metalloproteinase inhibiting peptidomimetic compounds found in snake venom. With regard to these compounds, a further object of the present invention is their use as pharmaceutically active agents for their effectiveness in the inhibition of metalloproteinases of endogenous origin produced by the organism of mammals, including man, and which cause a series of diseases.

The new compounds according to the invention can be described using the following general formula:
(a) 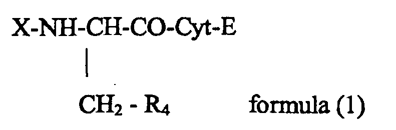
where:
E indicates OH, NH₂, NHOH, N(CH₃)OH or esters;
R₄ is CH-(CH₃)₂, indol-2-yl, phenyl, cicloheoyl, or
(CH₂)₃-NH-Fmoc;
X may be Xa, 5-methoxy-1-indanone-3-acetyl, naphtoyl, or homoseryl, where Xa is 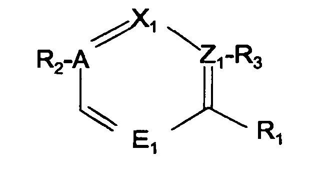 wherein
X₁ is : CH, N, or C-OMe
A is : C, or N
E₁ is : CH, or N
Z₁ is : C, or N
R₁ is : CO, (CH₂)₂-CO, SO₂, CH₂-CO, or S-CH₂-CO
R₂ is : OMe, H, NO₂, Cl, OEt, or CH₃
R₃ is : OEt, H, OMe
and pharmaceutically acceptable salts, esters and amides of the compounds listed above.

Description of the Chemical Synthesis of the Compounds.
Abbreviations used for REAGENTS and SOLVENTS:
**HBTU =** O-Benzotriazol-1-yl-N,N,N',N-Tetramethyluronium esafluorophosphate
**TEA** = Triethylamine
**SOCl**_{**2**} = Thionyl chloride
**DCHA =** Dicyclohexalamine
**DMAP =** Dimethylaminopyridine
**DMF** = Dimethylformamide
**DCC** = Dicycloesylcarbodiimide
**HOBT =** 1-Hydroxybenzotriazole
**TMSCl =** Trimethylchloroxylane
**CH**_{**2**}**Cl**_{**2**} = Dichloromethane
**CH**_{**3**}**CN =** Acetonitryl
**Cyt** is a residue of Cyclotriptophane
- **Boc-(L)-Leu-OH** = N-(tert-Butoxycarbmyl)-(L)-Lzu-OR
- **Boc-(L)-Trp-OH** = N-(tert-Butoxycarbonyl)-(L)-Trp-OH
- **Boc-(L)-Phe-OH** = N-(tert-Butoxycarbonyl)-(L)-Phe-OH
- **Boc-(L)-Cha-OH =** N-(tert-Butoxycarbonyl)-(L)-B-cyclohexyl-Ala-OH
- **Boc-(L)-Asn-OH =** N-(tert-Butoxycarbonyl)-(L)-Asn-OH
- **Boc-(L)-Lys(Fmoc)-OH** = Na-(tert-Butoxycarbonyl)-Ne-(9-Fluornylmethoxycarbonyl)-(L)-Lys-OH
- **PIC** = Picolinyl
- **2-PMTA** = (2-Pyrimidylthio)acetyl
- **4-PTA** = (4-Pyridylthio)acetyl
- **3-APZC** = (3-Amino-2-pyrazinyl)carbonyl
- **7-MBF** = 7-Methoxy-2-benzofuroyl
- **4-MQC** = (4-Methoxy-2-quinolyl)carbonyl
- **5-HIC** = (5-hydroxy-indole-2-yl)carbonyl
- **5-MIC** = (5-Methoxy-indole-2-yl)carbonyl
- **2-FUR** = 2-Furoyl
- **3-FUR** = 3-Furoyl
- **2-BZF** = 2-Benzofuroyl
- **2-QIC** = Quinaldyl
- **2-PZC** = Pyrazinoyl
- **2-MPA** = 2-Methoxyphenylacetyl
- **2-EBZ** = 2-Ethoxybenzoyl
- **5-MPZ** = (5-Methyl-Pyrazine-2-yl)carbonyl
- **6-MNC** = 6-Methylnicotinoyl
- **5-MIA** = 5-Methoxy-1-inadone-3-acetyl
- **2,4-DMB** = 2,4-Dimethoxybenzoyl
- **4-MBZ** = 4-Methoxybenzoyl
- **4-NBZ** = 4-Nitrobenzoyl
- **4-CBZ** = 4-Chlorobenzoyl
- **3-NIC** = Nicotinoyl
- **4-NIC** = Isonicotinoyl
- **3,4-DMB** = 3,4-Dimethoxybenzoyl
- **HDC** = 3-Phenylpropionyl
- **BZS** = Benzensulphonyl
- **4-EBZ** = 4-Ethoxybenzoyl
- **1-NAF** =1 Naphtoyl

Inhibitor compounds present invention and in the therapeutic uses that will be described below, the being described and published in the US patent No. 5,504,071 mentioned above are : **Compounds of formula (2):**

Q-B-T formula (2)

wherein Q is a monovalent radical of a ring molecule selected from the group consisting of 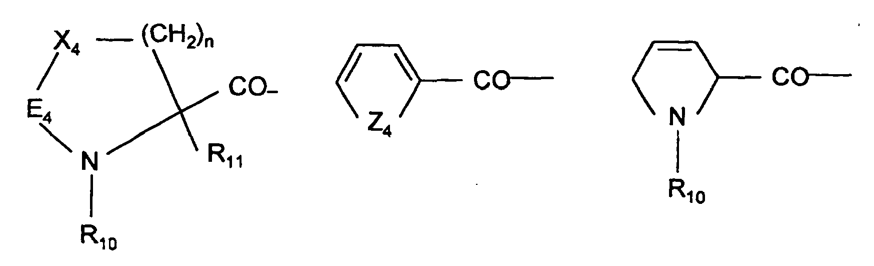 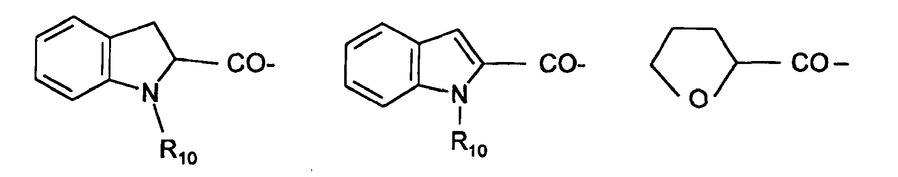 wherein
X₄ is CH₂, S or CHOH;
n is 0, 1 or 2;
R₁₁ is H or CH₃;
R₁₀ is H, CH₃ or a group used in peptide synthesis that blocks 1' nitrogen atom;
E₄ is CH₂ or CO; and
Z₄ is NH, NCH₃, O or S
B is a bivalent radical of an (L)-alpha-amino acid selected from the group glycine, leucine, alanine or valine; and
T is a monovalent radical of an (L)-amino acid selected from tryptophan, phenylalanine, phenylglycine;
and 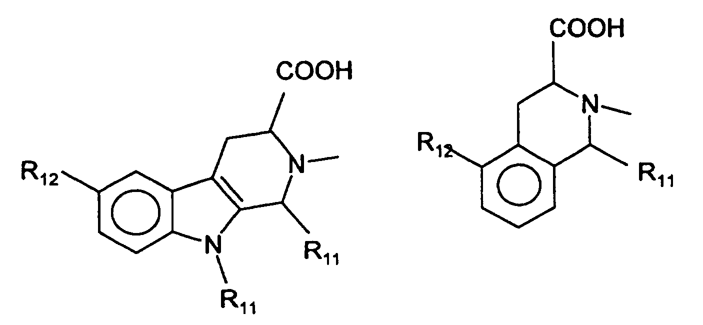 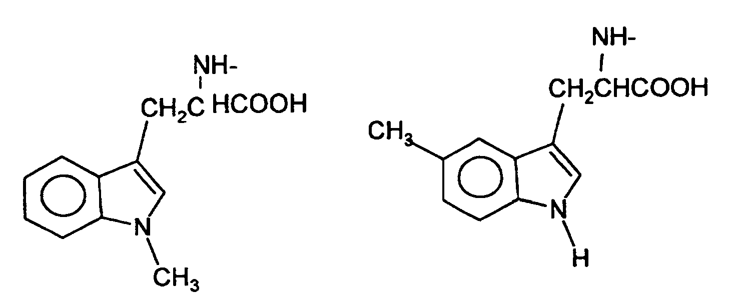 where R₁₁ is H or CH₃ ; and
R₁₂ is h, OH, OCH₃, OR CH₃ ; and
pharmaceutically acceptable salts, esters and amides of all the compounds listed above.

### EXAMPLE 1

### General process for synthesis of the compounds: X-(L)-A(R₄)-(S)-Cyt-Y

The general synthesis process comprises a first step, in which the intermediate compound (L)-A(R₄)-(S)-Cyt-Y is obtained. To this the residue X is subsequently added, using three different synthesis methods.

### I. Synthesis of the intermediate compound (L)-A(R₄)-(S)-CytOMe.

(S)-CytOMe.HCl (1.0 eq) and Boc-A(R₄)-OH (1.1 eq) are solubilized in CH3CN and cooled in an ice bath. They are then stirred for a few minutes, HBTU (1.2 eq) and TEA (2.2 eq) are added, and the mixture is kept under stirring until it reaches room temperature.

After this the reaction is concentrated on rotavapor and additioned with CH₂Cl₂. Washing is carried out using normal acid-basic treatments. The organic extracts are placed on anhydrous sodium sulphate and then cooled. They are dried and the product obtained is placed under vacuum on KOH for one night.

Boc-(L)-A-(R₄)-(S)-CytOMe (1,0 eq) are taken up with anhydrous dioxane and the solution, under stirring, is then cooled to 0° C and brought into an argon atmosphere. Once the temperature has stabilized, HCl 4M (4 eq) in anhydrous dioxane are added dropwise, allowing the temperature of the system to come up to room temperature, maintaining the mixture under stirring and in an argon atmosphere.

The product is concentrated on rotavapor with anhydrous ethyl ether without peroxides. The residue is taken up with nethanol and then precipatated with anhydrous ethyl ether without peroxides. It is then left at room temperature under stirring and then in a cool room.

The precipitate is filtered on G4 and placed under vacuum on P₂O₅.

(L)-A-(R₄)-(S)-CytOMe is obtained.

### II. Method a) for synthesis of the compounds X-(L)-A(R₄)-(S)-Cyt-OH.

(L)-NH2-CH(CH2-R4)-CO-(S)-CytOMe.HCl (1.0 eq) is added to the solution of X-OH acid (1.2 eq) in anhydrous CH₃CN. The solution is cooled in an ice bath under stirring and then HBTU (1.2 eq) and TE (2.2 eq) are added.

The reaction is made to proceed until completed. The reaction mixture is concentrated, CH₂Cl₂ is added, and it is then treated using normal acid-basic washing. The organic extracts are placed on anhydrous sodium sulphate and then cooled.

The product X-(L)-NH-CH(CH2-R4)-CO-(S)-CytOMe (1.0 eq) is taken up in CH₃CN and H₂O. The solution is cooled in an ice bath under stirring and NaOH 0.1 N (2.0 eq) is added. The solution is made to react for several hours, allowing the temperature to rise to room temperature. The reaction product is purified on SPE C18.

X-(L)-NH-CH(CH2-R4)-CO-(S)-CytOH is obtained.

### III. Method b) for synthesis of the compounds X-(L)-NH-CH(CH2-R4)-CO-(S)-CytOH

Pyridine (1.17 eq) and SOCl₂ (1.165 eq) are added under an argon flow and under stirring to the solution of X-OH acid DCHA salt (1.0 eq) in anhydrous CH₂Cl₂. One minute after addition of the thionyl chloride this is additioned with (L)-NH2-CH(CH2-R4)-CO-(S)-CytOMe.HCl (0.604 eq) and DMAP (1.202 eq) in anhydrous CH₂Cl₂. On completion of the reaction AcOEt is added and the organic phase is washed with a saturated solution of NaCl and subsequently with 10% citric acid and then with 5% bicarbonate. Finally, the organic extracts are washed with water saturated solution of NaCl until reaching a neutral pH and then placed on anhydrous sodium sulphate and cooled.

The organic solution, containing X-(L)-NH-CH(CH2-R4)-CO-(S)-CytOMe, is dried and then hydrolyzed.

The ester X-(L)-NH-CH(CH2-R4)-CO-(S)-CytOMe (1.0 eq) is taken up in CH₃CN and H₂O. The solution is cooled in an ice bath under stirring and NaOH 0.1 N (2.0 eq) is added. The solution is made to react for several hours, allowing the temperature to rise to room temperature.

The reaction product is purified on SPE C18.

X-(L)-NH-CH(CH2-R4)-CO-(S)-CytOH is obtained.

### IV. Method c) for preparation of the compounds X-(L)-NH-CH(CH2-R4)-CO-(S)-CytOH.

(L)-NH2-CH(CH2-R4)-CO-(S)-CytOMe.HCl (1.0 eq) and X-OH (1.1 eq) are solubilized in CH₃CN and cooled in an ice bath. After stirring for a few minutes, HBTU (1.2 eq) and TEA (2.2 eq) are added and the mixture is left under stirring, allowing the temperature to rise to room temperature.

On completion of the reaction AcOEt is added and the organic phase is washed with a saturated solution of NaCl and subsequently with 10% citric acid and then with 5% bicarbonate. The organic extracts are finally washed with water saturated solution of NaCl until reaching neutral pH and then placed on anhydrous sodium sulphate and cooled.

The organic solution, containing X-(L)-NH-CH(CH2-R4)-CO-(S)-CytOMe, is dried out and then hydrolyzed.

The ester X-(L)-NH-CH(CH2-R4)-CO-(S)-CytOMe (1.0 eq) is taken up in CH₃CN and H₂O. The solution is cooled in an ice bath under stirring and NaOH 0.1 N (2.0 eq) is added. The solution is reacted for several hours, allowing the temperature to rise to room temperature.

The reaction product is purified on SPE C18.

X-(L)-NH-CH(CH2-R4)-CO-(S)-CytOH is obtained.

### V. Particular cases of synthesis.

### Synthesis of the compound PIC-(L)-Leu-(S)-Cyt-NHOH

To the solution of 43 mg of Pic-Leu-Cyt-OH (1.0 eq) in 4 ml of anhydrous methylene chloride are added, under stirring and at room temperature, 19 mg of HOBT (1.25 eq) and 20.5 mg of DCC (1.0 eq).

After 60 minutes the solution is filtered and to the filtrate is added a mixture in methylene chloride (3 ml) of 8.5 mg of hydroxylamine hydrochloride (1.02 eq) and 17 mcl of triethylamine (1.02 eq). This is stirred for eighteen hours at room temperature and then concentrated on rotavapor. 20 ml of water are then added and the mixture is acidified with HCl 2N until reaching pH 2. 10 ml of a saturated solution of sodium chloride are added, and the mixture is extracted twice with 20 ml of ethyl acetate. The combined organic extracts are washed with saturated solution of sodium chloride until neutral. The organic phase is then placed on anhydrous sodium sulphate and cooled. The organic extracts, after drying, are taken up with 5 ml of methanol. The solution obtained in this manner is acidified with HCl 2 N to precipitate the excess of starting compound. The solution is filtered and dried, then lyophilized. The following is obtained: Picolinyl-(L)-Leu-(S)-Cyt-NHOH (abbreviated to **PIC-(L)-Leu-(S)-Cyt-NHOH**) (yield 50%). (Compound with molecular weight 449.49; empirical formula C₂₄H₂₇N₅O₄; Melting point 130 C; Elementary composition: C=64.23 (theor.64.13), H=6.11 (theor. 6.05), N=15.47 (theor. 15.58).

By the same procedure used for synthesis of PIC-(L)-NH-CH(CH2-R4)-CO-(S)-Cyt-NHOH, in which -NH-CH(CH2-R4)-CO- is equivalent to (L)-Leu and PIC is equivalent to Picolinyl, the following product was prepared:

**PIC-(L)-Leu-(S)-Cyt-N-(CH**_{**3**}**)-OH** (Compound with molecular weight 463.51, empirical formula C₂₅H₂₉N₅O₄; Melting point 145 C; Elementary composition: C=64.81 (theor. 64.78), H=6.35 (theor. 6.31), N=15.08 (theor. 15.11).

### EXAMPLE 2

### Synthesis of the compound 5-MIC-(L)-Cha-(S)-Cyt-OH

### I. Synthesis of the intermediate compound: Methyl-(S)-1,2,3,4-tetrahydro-9H-pyrido[3,4-b]indole-3-carboxylate.

10g (1.0 eq) of (L)-Trp-OH and 6.1 ml of 40% formaldehyde (1.8 eq) were additioned to 120 ml of a mixture of water and sulphuric acid 0.1N (v/v 2:1) and the mixture was left under stirring for 16 hours at room temperature. At the end of this period the precipitate obtained was filtered on G4 and washed using cold water and dried under vacuum on P₂O₅ for one night. The product (S)-1,2,3,4 tetrahydro-9H-pyrido[3,4-b]indole-3-carboxylic acid, abbreviated to (S)-CytOH.H₂SO₄ (yield 80%), obtained in this way was used for the following step.
(S)-CytOH.H₂SO₄ equivalent to 8 grammes (1.0 eq) and 18.6 ml of chlorotrimethylsylane (abbreviated to TMSC1, 4.0 eq) were added to 90 ml of absolute methanol. The reaction was carried out under argon at room temperature for 30 minutes from addition of the TMSCl and then the temperature was brought to 55° C and allowed to reflux for one night, again under argon.
The reaction was concentrated under vacuum three times with ethere, anhydrous and free from peroxides.
The residue was taken up with 5 ml of methanol and precipitated with anhydrous ether.
The compound was cooled and filtered on G4. The product was dried on P₂O₅ for one night. (S)-Cyt-OMe-HCl was obtained (yield 87%).

### II. Synthesis of the intermediate product: (L)-Cha-(S)-CytOMe.

(S)-Cyt-OMe-HCl equivalent to 128 mg (1.0 eq) and N-(tert-Butoxycarbonyl)-L-β-cyclohexyl-Ala-OH (abbreviated to Boc-Cha) 108 mg (1.1 eq) were added to 10 ml of acetonitrile (CH₃CN) cooled in an ice bath, the mixture was stirred for a few minutes and 181 ml of O-Benzotriazol-1-yl-N.N,N',N'-tetramethyluronium hexafluorophosphate (abbreviated to HBTU) (1.2 eq) and 122 mcl of Triethylamine (d=0.726, 2.2 eq) were added and the whole was left under stirring for 4 hours, allowing the temperature to come up to room temperature.
At the end of this time, the reaction was concentrated on rotavapor until reaching a volume of 8 ml and additioned with 90 ml of dichloromethane (CH₂Cl₂).
This was then washed in 80 ml of brine, washed twice in 80 ml of 4% hydrogen potassium sulphate. It was then washed three times with 80 ml brine until neutral and washed twice with 90 ml of 5% bicarbonate. It was washed until reaching neutral pH using a salt-saturated solution. The organic extracts were placed on anhydrous sodium sulphate and then cooled.
The resulting compound was dried and the foam obtained was placed under vacuum on KOH for one night.
The residue Boc-Cha-(S)-CytOMe was taken up with 8 ml of anhydrous dioxane and the solution, under stirring, was then cooled to 0°C and brought into an argon atmosphere. Once temperature had stabilized, ml 3.1 of HCl 4M in anhydrous dioxane were added dropwise, allowing the temperature of the system to reach room temperature, maintaining stirring and argon atmosphere for 4 hours.
The product was concentrated on rotavapor three times with anhydrous ether without peroxides.
The residue was taken up with 3 ml of methanol and then precipitated with 500 ml of anhydrous ether without peroxides.
It was then left for one hour at room temperature under stirring, and then for three hours in a coolroom.
The precipitate was filtered on G4 and placed under vacuum on P₂O₅.

Using the same procedure used for synthesis of (L)-NH2-CH(CH2-R4)-CO-(S)-CytOMe,
in which -NH-CH(CH2-R4)-CO- is equal to (L)-Cha, the following products were prepared:
HCl.(L)-Trp-(S)-Cyt-OMe
HCl.(L)-Phe-(S)-Cyt-OMe
HCl.(L)-Leu-(S)-Cyt-OMe
HCl.(L)-Asn-(S)-Cyt-OMe
HCl.(L)-Lys(Fmoc)-(S)-Cyt-OMe

### III. Synthesis of the compound 5-MIC-Cha-(S)-Cyt-OH according to Method a).

To the solution of mg 100 5-Methoxy-2-indolcarboxylic acid (Aldrich) (1.1 eq, abbreviated to 5-MIC) in 15 ml of anhydrous Acetonitrile (CH₃CN), cooled in an ice bath and under stirring, were added 200 mg of Cha-(S)-Cyt-OMe.HCl (1.2 eq). 217 mg of HBTU (Aldrich) (1.2 eq) and 150 mcl of Triethylamine (d=0.726, 2.2 eq) were then added.
The reaction was continued for three hours. At the end of this time the mixture was concentrated and 90 ml of methylene chloride were added, after which it was treated using normal acid-basic washing processes.
The organic extracts were placed on anhydrous sodium sulphate and then cooled.
250 mg of product 5-MIC-Cha-Cyt-OMe (1.0 eq) were taken up in 10 ml of acetonitrile and 0.7 ml of water. The solution was cooled in an ice bath under stirring
and then 9.3 ml of NaOH 0.1 N (2.0 eq) were added. The solution was stirred for 12 hours, allowing it to rise to room temperature. The reaction was treated in the following manner:

### 1) purification from the non-hydrolyzed ester

The reaction solution was loaded onto a 1 g C 18 SPE column (Backer), previously buffered with reaction solvent. The eluate containing only the product 5-MIC-Cha-(S)-CytOH was acidified by the addition of HCl 1N and additioned with water until the solution became torbid.

### 2) Purification of the acid

The solution containing the product was subsequently loaded onto 2 SPE columns (Backer) conditioned with the sample acid eluant. The eluate was eliminated and the SPE columns were eluted with methanol.
The combined organic phases were concentrated until reaching a volume of three ml and precipitated with 500 ml of HCl 0.1N.
After cooling in a cool chamber, the precipitate was filtered on G4 and under vacuum on P₂O₅ (yield 60%).
The compound **5-MIC-Cha-(S)-Cyt-OH (Compound 1)** was obtained.

### EXAMPLE 3

Using the same procedure used for synthesis of 5-MIC-(L)-A-(R₄)-(S)-Cyt-OH, where A-(R₄) is equal to (L)-Cha and 5-MIC is equal to (5-Methoxy-indol-2-yl)carbonyl, the following products were prepared:

**2-PMTA-(L)-Leu-(S)-Cyt-OH** **(Compound 2)**

**4-PTA-(L)-Len-(S)-Cyt-OH** **(Compound 3)**

**3-APZC-(L)-Leu-(S)-Cyt-OH** **(Compound 4)**

**7-MBF-(L)-Leu-(S)-Cyt-OH** **(Compound 5)**

**4-MQC-(L)-Leu-(S)-Cyt-OH** **(Compound 6)**

**5-HIC-(L)-Leu-(S)-Cyt-OH** **(Compound 7)**

**5-MIC-(L)-Phe-(S)-Cyt-OH** **(Compound 8)**

**PIC-(L)-Leu-(S)-Cyt-OH** **(Compound 9)**

**2-FUR-(L)-Cha-(S)-Cyt-OH** **(Compound 10)**

**2-FUR-(L)-Phe-(S)-Cyt-OH** **(Compound 11)**

**(L)-HomoSer-(L)-Leu-(S)-Cyt-OH** **(Compound 12)**

**TABLE 1. Chemical and physical characteristics of the compounds synthesized using the method described.**

| **No.** | **Mol.** | **Formula unit** | **Carbon %** | | **Hydrogen %** | | **Nitrogen %** | | **Melting** |
|---|---|---|---|---|---|---|---|---|---|
| | **Weight** | | **Found** | **Theor.** | **Found** | **Theor.** | **Found** | **Theor.** | **Point°C)** |
| 1 | 542.61 | C₃₁H₃₄N₄O₅ | 68.69 | 68.61 | 6.38 | 6.32 | 10.38 | 10.33 | 151 |
| 2 | 481.49 | C₂₄H₂₇N₅O₄S | 60.11 | 59.86 | 5.71 | 5.65 | 14.1 | 14.55 | 90 |
| 3 | 480.50 | C₂₅H₂₈N₄O₄S | 62.52 | 62.49 | 5.91 | 5.87 | 11.61 | 11.66 | 153 |
| 4 | 450.48 | C₂₃H₂₆N₆O₄ | 61.41 | 61.32 | 5.88 | 5.82 | 18.61 | 18.66 | 149 |
| 5 | 503.54 | C₂₈H₂₉N₃O₆ | 66.84 | 66.78 | 5.87 | 5.80 | 8.29 | 8.35 | 161 |
| 6 | 514.55 | C₂₉H₃₀N₄O₅ | 67.76 | 67.69 | 5.92 | 5.88 | 10.76 | 10.89 | 163 |
| 7 | 488.52 | C₂₇H₂₈N₄O₅ | 66.42 | 66.38 | 5.82 | 5.78 | 11.38 | 11.47 | 166 |
| 8 | 536.56 | C₃₁H₂₈N₄O₅ | 69.45 | 69.39 | 5.32 | 5.26 | 10.36 | 10.44 | 136 |
| 9 | 434.47 | C₂₄H₂₆N₄O₄ | 66.44 | 66.34 | 6.09 | 6.03 | 12.73 | 12.90 | 128 |
| 10 | 463.51 | C₂₆H₂₉N₃O₅ | 67.43 | 67.37 | 6.39 | 6.31 | 8.98 | 9.07 | 136 |
| 11 | 457.46 | C₂₆H₂₃N₃O₅ | 68.34 | 68.26 | 5.16 | 5.07 | 9.01 | 9.19 | 150 |
| 12 | 430.48 | C₂₂H₃₀N₄O₅ | 61.42 | 61.38 | 7.04 | 7.02 | 12.98 | 13.01 | 163 |

### EXAMPLE 4

### Synthesis of the compound 3-Fur-(L)-Leu-(S)-Cyt-OH according to Method b).

To the solution of 120 mg of 3-Furoic acid DCHA salt (1.0 eq) in 5 ml of anhydrous CH2Cl2 were added 39 mcl of pyridine (on potassium hydroxide) (1.17 eq) and 35 mcl of SOCl2 (1.165 eq) under an argon flow and under stirring. After one minute from addition of the thionyl chloride, 100 mg of (L)-Leu-(S)-CytOMe.HCl (0.604 eq) and 64 mg of DMAP (1.202 eq) in 3 ml of anhydrous CH2Cl2 were added.
After one hour, 80 ml of ethyl acetate were added and the organic phase was washed with a saturated solution of NaCl and subsequently with 10% citric acid (80*2 ml) and then with 5% bicarbonate (80*2 ml).
The organic extracts were then washed with a saturate water solution of NaCl (80*3 ml) until reaching a neutral pH and then placed on anhydrous sodium sulphate and cooled.
The organic solution, containing 3-Furoyl-(L)-Leu-(S)-Cyt-OMe, was dried and then hydrolyzed.
118 mg of the product 3-Furoyl-Leu-(S)-Cyt-OMe (1.0 eq) were taken up in 10 ml of CH3CN and 4.8 ml of H2O. The solution was cooled in an ice bath under stirring and then 5.2 ml of NaOH 0.1 N (2.0 eq) were added. The solution was stirred for 12 hours and then allowed to rise to room temperature.
The reaction was treated as follows:

### 1) Purification from the non-hydrolyzed ester

The reaction solution was loaded onto a 1 g C 18 SPE column (Backer), previously buffered with reaction solvent. The eluate containing only the product 3-Furoyl-(L)-Leu-(S)-Cyt-OH, was acidified by the addition of HCl 1N and additioned with water until the solution became torbid.

### 2) Purification of the acid

The solution containing the product was subsequently loaded onto 2 SPE columns (Backer) conditioned with the sample acid eluant. The eluate was eliminated and the SPE columns were eluted with methanol.
The combined organic phases were concentrated until reaching a volume of 3 ml and precipitated with 500 ml of HCl 0.1N.
After cooling in a cool chamber, the precipitate was filtered on G4 and placed under vacuum on P2O5.
The compound 3-Furoyl-(L)-Leu-(S)-Cyt-OH was obtained (abbreviated to **3-Fur-(L)-Leu-(S)-Cyt-OH)** (yield 60%) **(Compound 13).**

### EXAMPLE 5

Using the same process used for synthesis of 3-FUR-(L)-NH2-CH(CH2-R4)-CO-(S)-CytOMe, in which -NH-CH(CH2-R4)-CO- is equal to (L)-Leu and 3-FUR is equal to 3-Furoyl, the following products were prepared:

**5-MIC-(L)-Trp-(S)-Cyt-OH** **(Compound 14)**

**2-BZF-(L)-Leu-(S)-Cyt-OH** **(Compound 15)**

**2-QIC-(L)-Leu-(S)-Cyt-OH** **(Compound 16)**

**5-MIC-(L)-Leu-(S)-Cyt-OH** **(Compound 17)**

**2-PZC-(L)-Leu-(S)-Cyt-OH** **(Compound 18)**

**2-MPA-(L)-Leu-(S)-Cyt-OH** **(Compound 19)**

**2-EBZ-(L)-Leu-(S)-Cyt-OH** **(Compound 20)**

**5-MPZ-(L)-Leu-(S)-Cyt-OH** **(Compound 21)**

**6-MNC-(L)-Leu-(S)-Cyt-OH** **(Compound 22)**

**5-MIA-(L)-Leu-(S)-Cyt-OH** **(Compound 23)**

**TABLE 2 Chemical and physical characteristics of the compounds synthesized using the method described.**

| **No.** | **Mol. Weight** | **Formula unit** | **Carbon %** | | **Hydrogen %** | | **Nitrogen %** | | **Melting** |
|---|---|---|---|---|---|---|---|---|---|
| | | | **Found** | **Theor.** | **Found** | **Theor.** | **Found** | **Theor.** | **Point °C)** |
| 13 | 423.45 | C₂₃H₂₅N₃O₅ | 65.31 | 65.23 | 6.0 | 5.95 | 9.89 | 9.92 | 130 |
| 14 | 575.59 | C₃₃H₂₉N₅O₅ | 68.91 | 68.86 | 5.12 | 5.08 | 12.11 | 12.17 | 174 |
| 15 | 473.50 | C₂₇H₂₇N₃O₅ | 68.52 | 68.48 | 5.79 | 5.75 | 8.81 | 8.87 | 155 |
| 16 | 484.53 | C₂₈H₂₈N₄O₄ | 69.51 | 69.40 | 5.85 | 5.82 | 11.44 | 11.56 | 161 |
| 17 | 502.54 | C₂₈H₃₀NaO₅ | 67.03 | 66.92 | 6.06 | 6.02 | 11.08 | 11.15 | 158 |
| 18 | 435.46 | C₂₃H₂₅N₅O₄ | 63.50 | 63.43 | 5.81 | 5.79 | 16.10 | 16.08 | 132 |
| 19 | 477.54 | C₂₇H₃₁N₃O₅ | 67.91 | 67.90 | 6.60 | 6.49 | 8.76 | 8.80 | 133 |
| 20 | 477.54 | C₂₇H₃₁N₃O₅ | 67.98 | 67.90 | 6.59 | 6.49 | 8.75 | 8.79 | 128 |
| 21 | 449.49 | C₂₄H₂₇N₅O₄ | 64.21 | 64.13 | 6.10 | 6.05 | 15.48 | 15.58 | 151 |
| 22 | 448.50 | C₂₅H₂₈N₄O₄ | 67.02 | 66.95 | 6.34 | 6.29 | 12.39 | 12.49 | 159 |
| 23 | 530.57 | C₃₀H₃₂N₃O₆ | 67.98 | 67.91 | 6.12 | 6.08 | 7.87 | 7.92 | 147 |

### EXAMPLE 6

### Synthesis of the compound 2,4-DMB-(L)-Leu-(S)-Cyt-OH according to Method c).

63.4 mg of 2,4-Dimethoxybenzoyc acid chloride (Aldrich) (1.2 eq) were added to 15 ml of dry, oxide-free CH2Cl2. The mixture was stirred, and 100 mg of Leu-(S)-Cyt-OMe.HCl (1.0 eq) were added and 81 mcl of TEA (2.2 eq) were added. The solution was then placed in an ice bath under stirring for 2.5 hours and then 90 ml of CH2Cl2 were added. The solution was washed with 80 ml of saturate NaCl solution and then with 50 ml of HCl 0.1 N and then three times with 80 ml of saturated NaCl solution until reaching a neutral pH.
The organic extracts were placed on anhydrous sodium sulphate in a cold room.
The organic solution, containing 2,4-Dimethoxybenzoyl-Leu-(S)-Cyt-OMe, was dried (yield 95%) and then hydrolyzed.
133 mg of ester (1.0 eq) were taken up with 19 ml of CH3CN and 11.5 ml of H2O and the solution was cooled in an ice bath and then additioned with 5.23 ml of NaOH 0.1 N (2.0 eq).
The solution was stirred for six hours, allowing the temperature to rise to room temperature.
At the end of this time the reaction was treated as follows:

### 1) Purification from the non-hydrolyzed ester

The reaction solution was loaded onto a 1 g C 18 SPE column (Backer), previously buffered with reaction solvent. The eluate containing only the product 2,4-Dimethoxybenzoyl-Leu-(S)-Cyt-OMe, was acidified by the addition of HCl 1N and additioned with water until the solution became torbid.

### 2) Purification of the acid

The solution containing the product was subsequently loaded onto 2 SPE columns (Backer) conditioned with the sample acid eluant. The eluate was eliminated and the SPE columns were eluted with methanol.
The combined organic phases were concentrated until reaching a volume of 3 ml and precipitated with 500 ml of HCl 0.1N.
After cooling in a cool chamber, the precipitate was filtered on G4 and placed under vacuum on P2O5.
The compound 2,4-Dimethoxybenzoyl-(L)-Leu-(S)-Cyt-OH was obtained (abbreviated to **2,4-DMB-(L)-Leu-(S)-Cyt-OH)** (yield 74%) **(Compound 24).**

### EXAMPLE 7

Using the same process used for synthesis of 3-FUR-(L)-NH2-CH(CH2-R4)-CO-(S)-CytOMe, in which -NH-CH(CH2-R4)-CO- is equal to (L)-Leu and 3-FUR is equal to 3-Furoyl, the following products were prepared:

**4-MBZ-(L)-Trp-(S)-Cyt-OH** **(Compound 25)**

**4-NBZ-(L)-Leu-(S)-Cyt-OH** **(Compound 26)**

**4-CBZ-(L)-Leu-(S)-Cyt-OH** **(Compound 27)**

**3-NIC-(L)-Leu-(S)-Cyt-OH** **(Compound 28)**

**4-NIC-(L)-Leu-(S)-Cyt-OH** **(Compound 29)**

**3,4-DMB-(L)-Leu-(S)-Cyt-OH** **(Compound 30)**

**HDC-(L)-Leu-(S)-Cyt-OH** **(Compound 31)**

**BZS-(L)-Leu-(S)-Cyt-OH** **(Compound 32)**

**4-EBZ-(L)-Leu-(S)-Cyt-OH** **(Compound 33)**

**2-FUR-(L)-Trp-(S)-Cyt-OH** **(Compound 34)**

**2-FUR-(L)-Asn-(S)-Cyt-OH** **(Compound 35)**

**2-FUR.-(L)-Lys(Fmoc)-(S)-Cyt-OH** **(Compound 36)**

**1-NAF-(L)-Leu-(S)-Cyt-OH** **(Compound 37)**

**TABLE 3 Chemical and physical characteristics of the compounds synthesized using the method described.**

| **No.** | **Mol. Weight** | **Formula unit** | **Carbon %** | | **Hydrogen %** | | **Nitrogen %** | | **Melting Point °C)** |
|---|---|---|---|---|---|---|---|---|---|
| | | | **Found** | **Theor.** | **Found** | **Theor.** | **Found** | **Theor** | |
| 24 | 493.54 | C₂₇H₃₁N₃O₆ | 65.81 | 65.70 | 6.36 | 6.33 | 8.48 | 8.51 | 103 |
| 25 | 463.51 | C₂₆H₂₉N₃O₅ | 67.41 | 67.37 | 6.32 | 6.31 | 9.01 | 9.07 | 110 |
| 26 | 478.48 | C₂₅H₂₆N₄O₆ | 62.81 | 62.75 | 5.51 | 5.48 | 11.66 | 11.71 | 167 |
| 27 | 467.93 | C₂₅H₂₆N₃O₄Cl | 64.22 | 64.17 | 5.69 | 5.60 | 8.89 | 8.98 | 175 |
| 28 | 434.47 | C₂₄H₂₆N₄O₄ | 66.54 | 66.34 | 6.12 | 6.03 | 12.55 | 12.90 | 187 |
| 29 | 434.47 | C₂₄H₂₆N₄O₄ | 66.57 | 66.34 | 6.13 | 6.03 | 12.65 | 12.90 | 170 |
| 30 | 493.54 | C₂₇H₃₁N₃O₆ | 65.83 | 65.70 | 6.38 | 6.33 | 8.47 | 8.51 | 154 |
| 31 | 461.54 | C₂₇H₃₁N₃O₄ | 70.35 | 70.26 | 6.81 | 6.77 | 9.0 | 9.10 | 103 |
| 32 | 469.53 | C₂₄H₂₇N₃O₅S | 61.52 | 61.39 | 5.84 | 5.80 | 8.91 | 8.95 | 142 |
| 33 | 477.54 | C₂₇H₃₁N₃O₅ | 68.01 | 67.90 | 6.59 | 6.54 | 8.72 | 8.80 | 148 |
| 34 | 496.50 | C₂₈H₂₄N₄O₅ | 67.83 | 67.73 | 4.91 | 4.87 | 11.22 | 11.28 | 152 |
| 35 | 424.39 | C₂₁H₂₀N₄O₄ | 59.48 | 59.43 | 4.82 | 4.75 | 13.10 | 13.20 | 188 |
| 36 | 660.69 | C₃₈H₃₆N₄O₇ | 69.14 | 69.08 | 5.54 | 5.49 | 8.38 | 8.48 | 177 |
| 37 | 483.54 | C₂₉H₂₉N₃O₄ | 71.67 | 72.03 | 6.15 | 6.04 | 8.65 | 8.69 | 151 |

### DESCRIPTION OF THE BIOCHEMICAL ACTIVITY OF THE COMPOUNDS.

### EXAMPLE 8

### Inhibition of compounds on metalloproteinases produced from snake venom.

The compounds according to the present invention were tested on a number of zinc-dependent metalloproteinases extracted from snake venom, belonging both to the Crotalidae and the Viperidae (for example, Hemorrhagins from Crotalus Atrox, Botropasin, Hemorrhagin from Agkistrodon Bilineatus, various Hemorrhagins from Echis Carinatus). The following is a description of the purification of the Hemorrhagin from Crotalus Adamanteus, used for preference in the screening tests because of its manageability.

One gramme of lyophilised venom (obtained from the American company Sigma Chemical) was dissolved in Tris-HCl buffer solution at pH 8.0 and loaded on a chromatographic column containing DEAE Sephadex (trademark) A-50 resin. Using as an eluant a gradient of the buffer, containing NaCl in a concentration of 0 to 1 M, 4 main peaks were obtained for the proteic fractions present in the venom. These were concentrated and desalinated, and then tested for the presence of metalloproteinases using the method described below. Fraction I, containing the majority of metalloproteinase activity, was then passed on a chromatographic column containing Sephadex (trademark) G-150 resin, and eluted with Tris-HCl buffer pH 7.5. Two main peaks were obtained, and the metalloproteinase activity is identified in the first of these. Further purification, to remove extraneous material, was carried out using Sephadex (trademark) G-75 resin. The enzyme purified in this way was lyophilized and preserved in a freezer unit until use.

The metalloproteinase activity was tested on the fluorimeter (Perkin Elmer LS 50 B), using as a substrate the fluorigenic compound 2-Aminobenzoyl-Alanyl-Gly-Leucyl-Alanyl-p-Nitrobenzylamide, produced by the company Bachem. Different quantities of the compounds synthesized were added to 5 micrograms of enzyme, in a system thermostatically set to 30 degrees, under stirring. Formation of fluorescent compounds was followed for 30 minutes (excitation: 320 nm; emission: 420 nm), and the curve was compared with the base line, obtained without the addition of the compounds.

**TABLE 4 Inhibitory activity of the compounds according to the present invention on the enzyme purified from the venom of Crotalus Adamanteus.**

| **Compound No.** | **IC**_{**50**} | **Compound No.** | **IC**_{**50**} | **Compound No.** | **IC**_{**50**} |
|---|---|---|---|---|---|
| 1 | 2.9x10⁻⁸ | 14 | 4.7x10⁻⁸ | 27 | 1.0x10⁻⁷ |
| 2 | 2.3x10⁻⁷ | 15 | 4.0x10⁻⁸ | 28 | 1.0x10⁻⁷ |
| 3 | 3.6x10⁻⁷ | 16 | 2.8x10⁻⁸ | 29 | 4.0x10⁻⁷ |
| 4 | 6.7x10⁻⁸ | 17 | 2.3x10⁻⁸ | 30 | 3.8x10⁻⁷ |
| 5 | 9.4x10⁻⁸ | 18 | 2.1x10⁻⁷ | 31 | 2.1x10⁻⁷ |
| 6 | 5.0x10⁻⁸ | 19 | 2.6x10⁻⁷ | 32 | 2.0x10⁻⁶ |
| 7 | 2.3x10⁻⁸ | 20 | 1.3x10⁻⁷ | 33 | 7.0x10⁻⁸ |
| 8 | 1.0x10⁻⁷ | 21 | 4.4x10⁻⁸ | 34 | 5.0x10⁻⁷ |
| 9 | 2.7x10⁻⁸ | 22 | 1.1x10⁻⁷ | 35 | 8.0x10⁻⁶ |
| 10 | 2.5x10⁻⁸ | 23 | 7.5x10⁻⁸ | 36 | 1.0x10⁻⁷ |
| 11 | 5.3x10⁻⁸ | 24 | 9.8x10⁻⁸ | 37 | 2.1x10⁻⁷ |
| 12 | 5.0x10⁻⁶ | 25 | 5.4x10⁻⁸ | | |
| 13 | 4.0x10⁻⁷ | 26 | 9.5x10⁻⁸ | | |

### EXAMPLE 9

The same method described above was used to test the chemical compounds of formula (2) described in the US patent No. 5,504,071 to which reference is made for identification of the compounds indicated in the following table 5. All the compounds were found to be good inhibitors of the enzyme, with an IC₅₀ of between 1x10⁻⁵ and 1x10⁻⁷ M.

**TABLE 5 Activity of certain of the compounds synthesized on various metalloproteinases purified from snake venom.**

| **Compound No.** | | **IC50** | | |
|---|---|---|---|---|
| | Crotalus Adamanteus | Echis Carinatus | Bothrops Atrox | Bothrops Jararaca |
| 3 | 3.6x10⁻⁷ | 2.4x10⁻⁵ | 1.2x10⁻⁵ | 6.5x10⁻⁶ |
| 5 | 9.4x10⁻⁸ | 8.9x10⁻⁶ | 4.1x10⁻⁶ | 3.7x10⁻⁶ |
| 9 | 2.7x10⁻⁸ | 3.5x10⁻⁶ | 2.5x10⁻⁶ | 1.3x10⁻⁶ |
| 15 | 4.0x10⁻⁸ | 2.0x10⁻⁶ | 8.2x10⁻⁶ | 1.4x10⁻⁵ |
| 18 | 2.1x10⁻⁷ | 7.0x10⁻⁶ | 5.2x10⁻⁶ | 8.4x10⁻⁶ |
| 22 | 1.1x10⁻⁷ | 1.4x10⁻⁵ | 3.2x10⁻⁶ | 2.1x10⁻⁵ |
| 31 | 2.1x10⁻⁷ | 1.8x10⁻⁵ | 2.6x10⁻⁵ | 4.2x10⁻⁶ |
| 33 | 7.0x10⁻⁸ | 9.0x10⁻⁷ | 7.8x10⁻⁶ | 5.0x10⁻⁶ |

Compound 31 is **Z-(L)-Pro-(L)-Ala-(S)-CytOH**
Compound 33 is **Z-(L)-Pro-(L)-Ala-(9)-Me-(S)-CytONM**
The results expressed in Tables 4 and 5 testify that the compounds described in the present application, and in the US patent No. 5,504,071, are all extremely active in inhibiting the enzyme purified from the venom of Crotalus Adamanteus, and also show a moderate inhibitory activity with respect to other metalloproteinases purified from the venom of other Crotalidae and Viperidae, so that they can be considered inhibitors of the whole class of snake venom metalloproteinases.
The following Examples 10, 11 and 12 show the inhibition activity of selected compounds with respect to some metalloproteinase enzymes endogenous in humans and mammals.

### EXAMPLE 10

### "In vitro" effects on recombinant human gelatinase A.

Assays on human gelatinase A, an enzyme released in high concentration by any kind of tumour cells during the processes of metastatization, and recognized also as a product of stimulated cells in many pathological processes, have been performed using the protein obtained by recombinant biotechnology (Strangeways Laboratories, Cambridge, UK). The enzymatic activity has been followed during the time, measuring the amount of fluorescent substrate cleaved by the enzyme, in the presence or absence of synthetic compounds (Perkin Elmer L 50 B fluorimeter).

**TABLE 6**

| **Compound** | **Concentration** | **% Inhibition** |
|---|---|---|
| This Patent application: n. 1 | 7x10⁻⁷ M | 25 |
| "n. 11 | 8.7x10⁻⁷ M | 40 |
| " n. 13 | 9.4x10⁻⁷ M | 41 |
| " n. 14 | 6.0x10⁻⁷ M | 37 |
| " n. 16 | 8.0x10⁻⁷ M | 23 |
| " n. 22 | 8.0x10⁻⁷ M | 47 |
| " n. 23 | 8.0 x 10⁻⁷ M | 40 |
| " n. 31 | 8.0x10⁻⁷ M | 30 |

| From PCT WO92/06108 | | |
|---|---|---|
| **Exam. 1: Z-PRO-LEU-CYT** | 1.8 x 10⁻⁶ M | 64 |
| **Exam. 8: FUR-LEU-TRP** | 1.0 x 10⁻⁵ M | 41 |
| **Exam. 8: Tiof-LEU-TRP** | 8.0 x 10⁻⁶ M | 60 |
| **Exam. 9: Pirr-LEU-TRP** | 9.0 x 10⁻⁶ M | 33 |
| **Exam. 10: MePRO-ALA-CYT** | 9.0 x 10⁻⁶ M | 39 |
| **Exam. 15: Z-Pip-LEU-TRP** | 8.0 x 10⁻⁶ M | 63 |

The obtained results indicate that compounds described in the present application, as well as those described in PCT WO92/06108, are rather powerful inhibitors both of snake venom hemorrhagic factors, and of human gelatinase A, an enzyme heavily involved in pathological disturbances.

### EXAMPLE 11

### "In vitro" effects on recombinant human gelatinase B.

Assays on human gelatinase B, another enzyme released by tumour cells, have been performed using also in this case the protein obtained by recombinant biotechnology (Strangeways Laboratories, Cambridge, UK). As usual, the enzymatic activity has been followed during the time, measuring the amount of fluorescent substrate cleaved by the enzyme, in the presence or absence of synthetic compounds (Perkin Elmer L 50 B fluorimeter).

**TABLE 7**

| **Compound** | **Concentration** | **% Inhibition** |
|---|---|---|
| This patent application | | |
| Compound n. 9 | 1.1x10⁻⁵ M | 20 |
| "n.10 | 1.1x10⁻⁵ M | 32 |
| "n.16 | 1.0x10⁻⁵ M | 51 |
| " n. 21 | 5.5x10⁻⁶ M | 43 |
| " n. 24 | 1.0x10⁻⁵ M | 48 |
| " n. 32 | 1.0x10⁻⁵ M | 42 |
| From PCT WO92/06108 | | |
| **Exam. 1: Z-PRO-LEU-CYT** | 9.0 x 10⁻⁶ M | 32 |
| **Exam. 8: FUR-LEU-TRP** | 1.0 x 10⁻⁵ M | 22 |
| **Exam. 10: MePRO-LEU-CYT** | 1.1 x 10⁻⁵ M | 37 |

The above results indicate that also gelatinase B, another enzyme produced by human cells during pathological reactions, can be inhibited by molecules described in the present application, and in PCT WO92/06108.

### EXAMPLE 12

### "In vitro" assays on recombinant human collagenase.

Using the same enzymatic determination already described (fluorimetric detection of cleavage of a substrate peptide containing a fluorescent moiety), some of the compounds have been tested on neutrophil collagenase, an enzyme described as participating to any kind of inflammatory reaction in mammalian bodies. Also in this case, the pure enzyme has been obtained through recombinant biotechnology.

**TABLE 8**

| **Compound** | **Concentration** | **% Inhibition** |
|---|---|---|
| This patent application | | |
| Compound n. 10 | 2.1 x 10⁻⁵ M | 51 |
| "n. 11 | 2.2x10⁻⁵ M | 27 |
| "n. 24 | 2.0x10⁻⁵ M | 19 |
| " n. 25 | 5.2x10⁻⁵ M | 23 |
| " n. 27 | 2.1x10⁻⁵ M | 28 |

| From PCT WO92/06108 | | |
|---|---|---|
| **Exam. 1: Z-PRO-LEU-CYT** | 1.8 x 10⁻⁵ M | 23 |
| **Exam. 8: FUR-LEU-TRP** | 2.4 x 10⁻⁵ M | 12 |
| **Exam. 9: Pirr-LEU-TRP** | 1.1 x 10⁻⁵ M | 15 |

Results indicate some kind of inhibitory activity also against one of the most important enzymes produced by human cells during inflammatory reactions.

### DESCRIPTION OF THE PHARMACOLOGICAL ACTIVITY OF THE COMPOUNDS.

### EXAMPLE 13

### "In vitro" activity on Tumour Necrosis Factor (TNF).

It is well known that the TNF plays a fundamental role in certain physiological defense responses, but on the other hand causes severe damage when it is released into circulation in excessive amounts or for prolonged periods. Recently, it was demonstrated that the TNF is produced in an active form on the cell surface thanks to a zinc-dependent metalloproteinase. An attempt was therefore made to see whether certain compounds selected from among those described in the present application and in US patent No. 5,504,071, were capable of blocking the release of active TNF in human cell culture medium (Jurkat). The cells were incubated in the presence or in the absence of metalloproteinase inhibitor for 72 hours; the release of TNF was then stimulated using activators (PMA and ionophore calcium), and the Factor was dosed into the culture medium using the Elisa test (Genzyme).

Compounds 9, 10 and 25 of the present invention all showed a dose-dependent inhibitor effect on TNF release by cells, although to a different extent (compound 9 showed an IC50 or around 8 mcg/ml; compound 10 around 200 mcg/ml; and compound 25 around 0.7 mcg/ml). Furthermore, the compound Z-PRO-LEU-Cyt (described in example 1 of the US patent No. 5,504,071) also showed itself to be active, with an IC50 of around 5 mcg/ml. This compound was also made to undergo another test to evaluate TNF release from another strain of human cells in culture (clone T): in this case also inhibitory activity was seen (IC50 lower than 1 mcg/ml).

These results demonstrate that snake venom metalloproteinase inhibitors are capable of blocking TNF release from human cells, and are therefore potentially useable for treatment in a number of pathological situations, which range from rheumatoide arthritis (and other forms of arthritis) to septic shock, to multiple sclerosis, to immunodeficiency caused by viral infections.

### EXAMPLE 14

### "In vivo" activity on TNF.

The same compounds used in the "in vitro" tests were also made to undergo tests to see whether they were capable of blocking mortality induced in mice of the strain Balb/c by means of lethal doses of LPS (a bacterial agent that stimulates the release of TNF). All the compounds showed themselves to be capable of preserving the mice from death, with an IC50 varying from 0.5 to 5 mcg/mouse (i.p. injection).

These results confirm the potential usability of the snake venom metalloproteinase inhibitors in all pathological situations caused by TNF.

### EXAMPLE 15

### Activity on human collagenase.

The compounds described in the present application, and those of the US patent No. 5,504,071, were tested on the enzyme collagenase purified from human neutrophiles. Over half of the compounds showed themselves capable of strongly inhibiting the enzyme, which is also known to be directly connected with inflammatory response (rheumatoide arthritis, osteoarthrosis, etc.), with tumoral invasion, cicatrisation of wounds, periodontitis, corneal ulcers, etc.

For this reason it can be stated that the inhibition test on snake venom metalloproteinases is highly predictive of an inhibitory activity on the collagenase produced by human cells, and makes it possible to predict important therapeutic activity in connection with the inhibition itself.

### EXAMPLE 16

### Inhibition of haemorrhages induced by snake venom.

The compound Fur-LEU-TRP (described as example 8 in the US patent No. 5,504,071), an excellent "in vitro" inhibitor of the metalloproteinase purified from the venom of Crotalus Adamanteus, was made to undergo "in vivo" testing on mice, to test its ability to antagonise the haemorrhages and lethal results induced by snake venoms. The results have shown that the compound, incubated with the haemorrhagic factors of various snake venoms, is capable of neutralising the toxic effects thereof. Furthermore, at a dose of 33 mcg/mouse the compound is capable of protecting the mice from death by injection of the venom, even when administered after the venom itself (up to 30 minutes).

Other compounds from the above mentioned US patent and from the present application are capable of antagonising the toxic and lethal effects of snake venoms (Crotalidae and Viperidae), although not so strongly.

It can therefore be stated that the snake venom metalloproteinase inhibitors form a new class of synthetic anti-venom drugs for use in case of poisoning by snakes of the families Crotalidae and Viperidae.

### EXAMPLE 17

### Effects on histamine-induced microhemorrhages.

The compound Z-PRO-LEU-Cyt, described as example 1 of the US patent No. 5,504,071, was made to undergo extensive investigations on the model of capillary permeability variation induced in hamsters following administration of histamine.

This model is generally useful to observe compounds with potential activity on the microcirculation and on atherosclerosis phenomena. Furthermore, as the administration of histamine induced microhemorrhages at arteriole level, in a manner similar to that seen in case of administration of snake venom haemorrhagic factors, using this model it is possible to evaluate whether or not the compounds being studied are capable of interacting with metalloproteinases endogenous in mammals, which are responsible for the effects of histamine.

The results underlined the fact that the compound is extremely powerful in blocking microhaemorrhages induced by histamine, both when injected intravenously, and when administered orally. In both cases, the dose capable of blocking 50% of effects was around 50 mcg/Kg.

Other compounds described in the present application and in the above mentioned US patent also showed themselves capable of inhibiting microhaemorrhages induced by histamine in hamsters, although at slightly higher concentrations.

It can therefore be deduced that the inhibitors of snake venom metalloproteinases are capable of antagonising the pathological processes that lead to internal microhaemorrhages, and are therefore potentially of use in arterosclerosis and in a number of other pathological conditions deriving from lesions of the vascular tissue.

### EXAMPLE 18

### Effects on infiltrations in the bronco-pulmonary tissue.

As the metalloproteinases produced by the cells of mammals are of fundamental importance to allow the cells to migrate from one tissue to another within the organism, compounds 9, 18, 25 and 31 described in the present application, together with the compounds Z-PRO-LEU-Cyt (example No. 1) and FUR-LEU-Cyt (described in example No. 8) of the US patent No. 5,504,071, were tested on hypereosinophilia induced in the rat by Sephadex G-200 resin. The results obtained demonstrate that all the compounds, although to varying extent, interfere with the number of blood cells that migrate to the inflamed site, inhibiting the motility of one or more of the classes of cell analysed (lymphocytes, neutrophiles, eosinophils, macrophages).

It can therefore be concluded that the snake venom metalloproteinase inhibitors are capable of acting on the motility of the cells involved in the inflammatory response at a bronco-pulmonary level, and may be of therapeutic use in the numerous bronco-pulmonary conditions caused by metalloproteinases, including pulmonary emphysema, adult respiratory deficiency syndrome (ARDS), interstitial fibrosis, granulomatose sickness, tumour of the lung and pleuritys.

### EXAMPLE 19

### Immunologic effects.

Many of the compounds described in the present application have been tested in immune response tests, to check whether they were capable of influencing progress of the response. The results obtained demonstrate that all the compounds are capable of stimulating the immune response, in a more or less marked manner, expressed as the proliferation of T lymphocytes. Furthermore, many of the compounds showed themselves capable of increasing the response to the influenza virus, determined by the presence of antibodies specific to that virus. Finally, in the contact sensitivity test (related to allergic response) it was found that many of the compounds were powerful inhibitors of the response.

It can therefore be concluded that snake venom metalloproteinase inhibitors are capable of significantly influencing the immune responses of mammals, both increasing the proliferation of T lymphocytes and increasing the production of antibodies. Furthermore, they are capable of blocking allergic type responses.

### EXAMPLE 20

### Inhibition of human melanoma cell invasion through matrigel.

In order to obtain informations about the possible interference of the synthesized compounds on the ability of tumour cells to migrate through the body (metastization), the human cell line melanoma VMM-5 has been used in the matrigel-based invasion assay, in which basal membrane matrigel (Becton Dickinson) and tissue culture inserts with 8 microns pore size in 24 well tissue culture plates were used as Boyden chambers. The chambers were coated with diluted matrigel and allowed to dry overnight; they were then reconstituted with serum free medium, and melanoma cells (400000/ml) were dispensed in each insert. In the lower chambers, a chemoattractant was included. The chambers were finally placed in humidified CO₂ incubator at 37 celsius degrees, and incubated for 20 hours. The inserts were dried and the cells on the upper surface removed. The polycarbonate filters were fixed with methanol, stained with hematoxylin and eosin, and counted with a microscope. Inhibition of migration due to compounds has been expressed as percentage of the total number migrated cells in the control chambers containing only the buffer in which the compounds have been dissolved.

**TABLE 9**

| **Compound** | **Concentration (microM)** | **% Inhibition** of invasion |
|---|---|---|
| This patent application | | |
| compound n. 3 | 500 | 69 |
| "n. 9 | 500 | 94 |
| "n. 10 | 500 | 99 |
| " n. 24 | 500 | 100 |

| From PCT WO92/06108 | | |
|---|---|---|
| **Exam. 1: Z-PRO-LEU-CYT** | 500 | 70 |
| **Exam. 8: FUR-LEU-CYT** | 500 | 69 |
| **Exam. 10: MePRO-LEU-CYT** | 500 | 40 |

The above results show that compounds described in the present application, as well as those reported in PCT WO92/06108, are strong inhibitors of cancer cells motility, and therefore can be utilized as inhibitors of metastization processes in patients affected by tumours.

### EXAMPLE 21

### Resistance to proteolytic activity in stomach juices.

A. To check if the described compounds are resistant to proteolytic activity in stomach juices, and therefore could be administered by oral route, stomach of fasted rats have been homogenized in physiological solutions (1:1). 300 microliters of solutions of compounds (1 mg/ml of saline) were incubated with 0.5 ml of stomach homogenates (37° C). The reaction was blocked through addition of 1.5 ml methanol and, after centrifugation, 10 microliters were injected in a HPLC apparatus against the standard solutions of compounds, in order to measure the amount of remaining compound.
Results:

**TABLE 10**

| **Compound (formula 1)** | **Residue after 60 minutes** | **Residue after 120 minutes** |
|---|---|---|
| n. 3 | 100% | >95% |
| n. 10 | 100% | >95% |
| n. 11 | 100% | >95% |
| n. 26 | 100% | >95% |
| n. 30 | 100% | >95% |

| | | |
|---|---|---|
| n. 33 | 100% | >95% |

B. To check whether the compounds of formula 2 were capable of resisting decay by hydrogen chloride and the proteolytic enzymes present in the stomach, some rats were kept fasting for approximately 18 hours, then sacrificed, and their stomach was homogenated in physiological solution (1:1). The compounds were solubilized, in physiological solution (1 mg/ml), and 300 microliters of compound were set to incubate with 0.5 ml of homogenate at 37 degrees C. The reaction was blocked at pre-set times by addition of 1.5 ml of methanol. After spinning for 15 minutes (Sorvall Centrifugal machine, rotorSS-34, 10,000 revs/minute), a small amount (10 microliters) was taken and injected into HPLC, to measure the amount of whole compound remaining. The Compound Number and the Example Number of preparation refer to PCT WO92/06108.

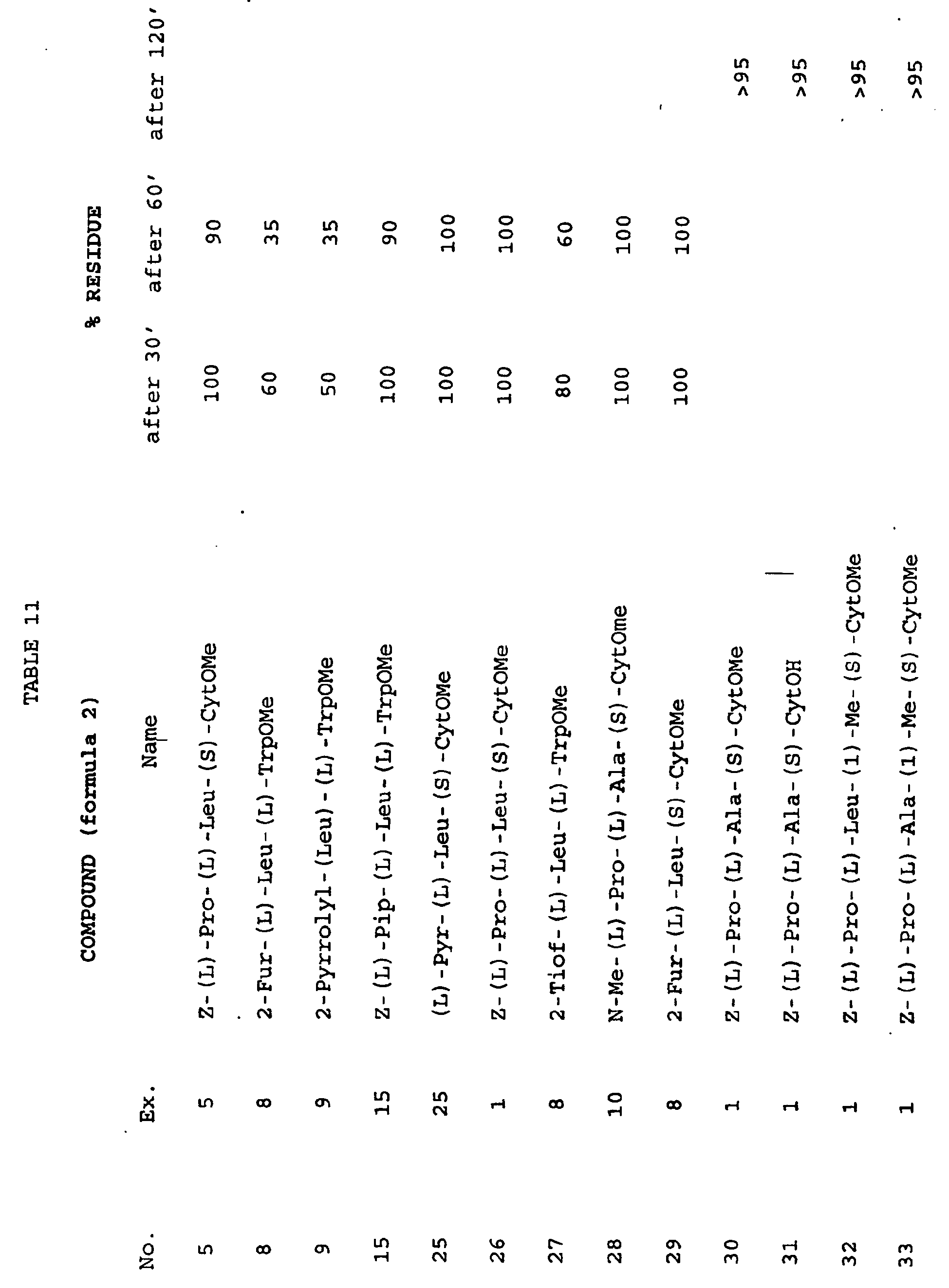

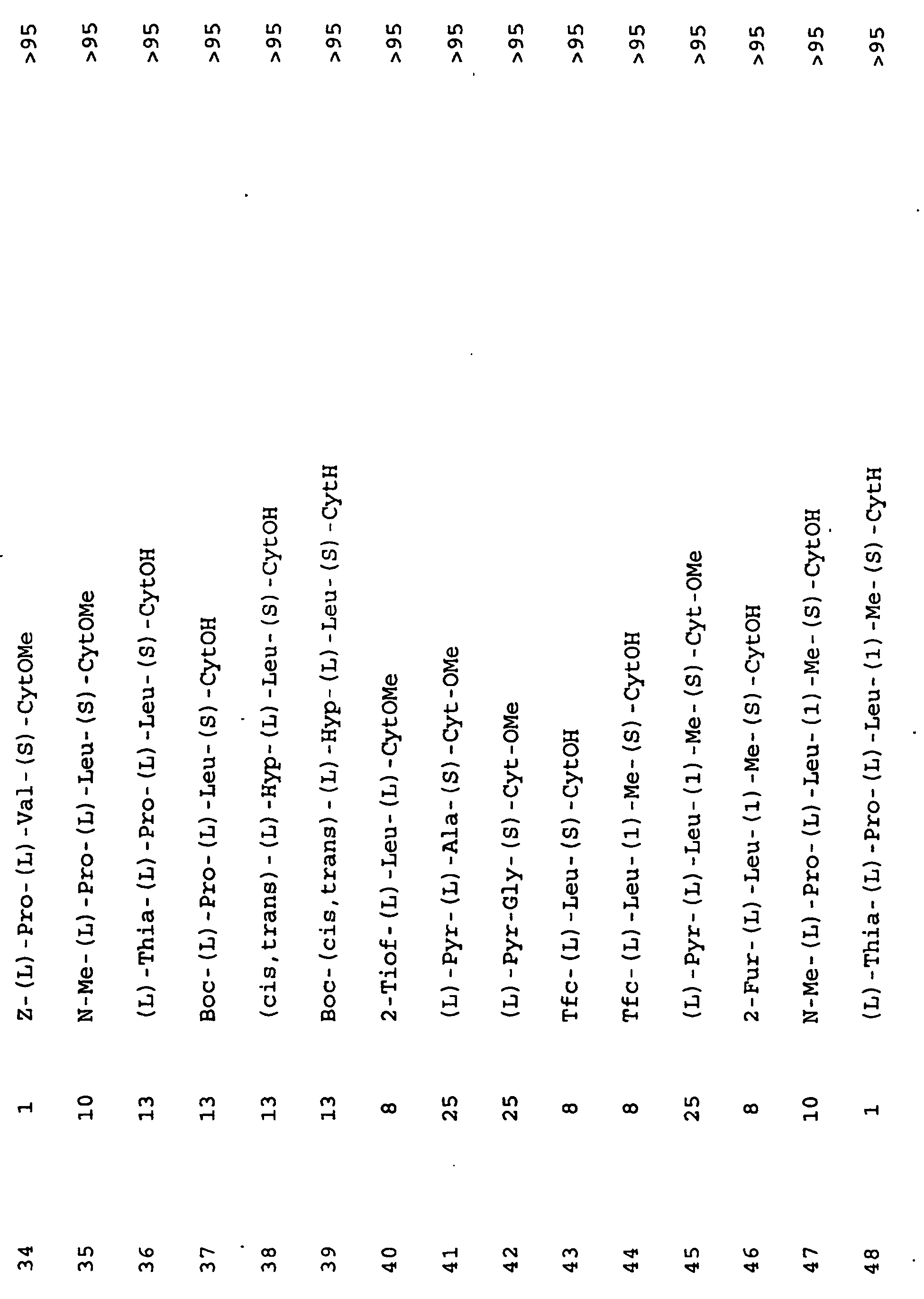

### LEGEND:

CytOH is the free acid form of the cyclotryptophan radical representative of group C in claim 12.
Hyp is a hydroxy proline radical representative of group A.
As can be clearly seen from this last table, the new series of compounds is extremely resistant to gastric decay, and are therefore capable of being used for oral administration. A suitable dayly dosage for humans, per OS, ranges from 10 to 200 mg of compound.

## Claims

1. Peptidomimetic compounds of formula
(a) 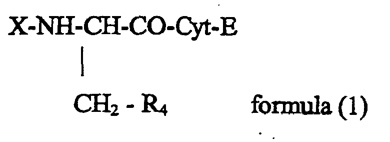
where:
E indicates OH, NH₂, NHOH, N(CH₃)OH or esters;
R₄ is CH-(CH₃)₂, indol-2-yl, phenyl, ciclohexyl, or
(CH₂)₃-NH-Fmoc;
Cyt- indicates a cyclotriptophan residue,
X is Xa, 5-methoxy-1-indanone-3-acetyl, naphtoyl, or homoseryl, where Xa is 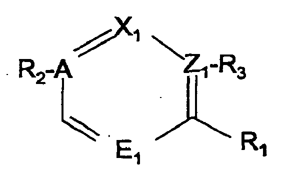
wherein
X₁ is : CH, N, or C-OMe
A is : C, or N
E₁ is : CH, or N
Z₁ is : C, or N
R₁ is : CO, (CH₂)₂-CO, SO₂, CH₂-CO, or S-CH₂-CO
R₂ is : OMe, H, NO₂, Cl, OEt, or CH₃
R₃ is : OEt, H, OMe
and pharmaceutically acceptable salts, esters and amides of all the compounds listed above.

2. A method for determining the therapeutic potential of a peptidomimetic compound as an inhibitor of zinc-dependent metalloproteinase activity associated with pathological conditions of humans and animals, comprising the steps of:
(a) conducting a first enzyme inhibition test and determining a first level of inhibitory activity of peptidomimetic compounds as inhibitors of a zinc-dependent metalloproteinase extracted from the venom of snakes belonging to the families Crotalidae and Viperidae;
(b) screening said compounds on the basis of said first level of inhibitory activity by assessing whether each of said compounds has an inhibitory activity on said zinc-dependent metalloproteinase from snake venom,
(c) conducting a second enzyme inhibition test and determining a second level of inhibitory activity of said compounds as inhibitors of zinc-dependent metalloproteinase endogenous in a mammal, the activity of said zinc-dependent metalloproteinase being associated with at least one pathological condition in said mammal;
(d) screening said compounds on the basis of said second level of inhibitory activity by assessing whether each of said compounds has an inhibitory activity on said zinc-dependent metalloproteinase endogenous in mammals; whereby
step (a) and step (b) make a primary selection of the tested compounds as inhibitors of said zinc-dependent metalloproteinase from snake venom and therefor as potential inhibitors of zinc-dependent metalloproteinases in mammals;
step (c) and step (d) test the capability of said selected compounds for inhibiting the zinc-dependent metalloproteinases associated with at least one pathological condition in mammals,
**characterized in that** said peptidomimetic compounds have the formula 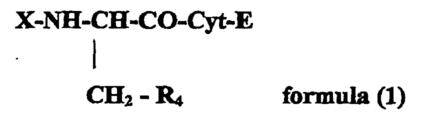 in which X, Cyt, E and R₄ have the same meaning as defined in claim 1.

3. A therapeutically active agent of formula 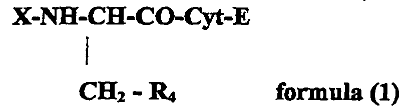 in which X, Cyt, E and R₄ have the same meaning as defined in claim 1, and pharmaceutically acceptable salts, esters and amides thereof, for the treatment of pathological conditions related to the activation of at least one Zn metalloproteinase from the class of Zn-dependent metalloproteinases endogenous in mammal tissues.

4. An agent according to claim 3, wherein said metalloproteinase endogenous in mammals is selected from gelatinase or collagenase,

5. An agent according to claim 4, wherein said pathological conditions associated with activation of gelatinase are processes of metastasization.

6. An agent according to claim 4, wherein said pathological conditions associated with activation of collagenase are inflammatory reactions, rheumatoid arthritis, osteoarthritis, tumoral invasion, wound healing, periodontitis, and corneal ulcers.

7. A therapeutically active agent according to any one of claims 3 to 6, in which said compound is selected from the group consisting of
PIC-(L)-Leu-(S)-Cyt-OH
2,4-DMB-(L)-Leu-(S)-Cyt-OH
5-MIC-(L)-Cha-(S)-Cyt-OH
5-MIC-(L)-Trp-(S)-Cyt-OH
2-QIC-(L)-Leu-(S)-Cyt-OH
6-MNC-(L)-Leu-(S)-Cyt-OH
5-MIA-(L)-Leu-(S)-Cyt-OH
HDC-(L)-Leu-(S)-Cyt-OH
BZS-(L)-Leu-(S)-Cyt-OH
4-MBZ-(L)-Trp-(S)-Cyt-OH
4-CBZ-(L)-Leu-(S)-Cyt-OH

8. A therapeutically active agent according to claim 3 for treating pathological conditions related to the release by the cells of mammals, including man, of the Tumor Necrosis Factor.

9. An active agent according to claim 8, wherein said compound is selected from PIC-(L)-Leu-(S)-Cyt-OH, or
4-MBZ-(L)-Trp-(S)-Cyt-OH.

10. An active agent according to claim 8 or 9, wherein said pathological conditions are septic shock, multiple sclerosis and immunodeficiency caused by viral infection.

11. A therapeutically active agent according to claim 1 for treating pathological conditions related to the toxic and lethal effects of snake venom

12. An active agent according to claim 11, wherein said compound is selected from the group consisting of
5-MIC-Cha-(S)-Cyt-OH
2-PMTA-(L)-Leu-(S)-Cyt-OH
4-PTA-(L)-Leu-(S)-Cyt-OH
3-APZC-(L)-Leu-(S)-Cyt-OH
7-MBF-(L)-Leu-(S)-Cyt-OH
4-MQC-(L)-Leu-(S)-Cyt-OH
5-HIC-(L)-Leu-(S)-Cyt-OH
5-MIC-(L)-Phe-(S)-Cyt-OH
PIC-(L)-Leu-(S)-Cyt-OH
(L)-HomoSer-(L)-Leu-(S)-Cyt-OH
5-MIC-(L)-Trp-(S)-Cyt-OH
2-BZF-(L)-Leu-(S)-Cyt-OH
2-QIC-(L)-Leu-(S)-Cyt-OH
5-MIC-(L)-Leu-(S)-Cyt-OH
2-PZC-(L)-Leu-(S)-Cyt-OH
2-MPA-(L)-Leu-(S)-Cyt-OH
2-EBZ-(L)-Leu-(S)-Cyt-OH
5-MPZ-(L)-Leu-(S)-Cyt-OH
6-MNC-(L)-Leu-(S)-Cyt-OH
5-MIA-(L)-Leu-(S)-Cyt-OH
2,4-DMB-(L)-Leu-(S)-Cyt-OH
4-MBZ-(L)-Trp-(S)-Cyt-OH
4-NBZ-(L)-Leu-(S)-Cyt-OH
4-CBZ-(L)-Leu-(S)-Cyt-OH
3-NIC-(L)-Leu-(S)-Cyt-OH
4-NIC-(L)-Leu-(S)-Cyt-OH
3,4-DMB-(L)-Leu-(S)-Cyt-OH
HDC-(L)-Leu-(S)-Cyt-OH
BZS-(L)-Leu-(S)-Cyt-OH
4-EBZ-(L)-Leu-(S)-Cyt-OH
1-NAF-(L)-Leu-(S)-Cyt-OH

13. An agent according to claim 11 or 12 wherein said pathological conditions are haemorrhages induced by snake venom.

14. An agent according to any one of claims 11 to 13, wherein said venom belongs to snakes of the Families Crotalidae and Viperidae.

15. A therapeutically active agent according to claim 3 for the treatment of pathological conditions that lead to internal haemorrhages of the vascular tissue.

16. An agent according to claim 15, wherein said pathological conditions are internal microhaemorrages, or arteriosclerosis and other pathological conditions deriving from lesions of the vascular tissue.

17. A therapeutically active agent according to claim 3 for treating pathological conditions related to the motility of the cells involved in the inflammatory response at a bronco-pulmonary level.

18. An active agent according to claim 17, wherein said compound is selected from the group consisting of
4-MBZ-(L)-Trp-(S)-Cyt-OH
HDC-(L)-Leu-(S)-Cyt-OH
2-PZC-(L)-Leu-(S)-Cyt-OH,
or
2-PZC-(L)-Leu-(S)-Cyt-OH

19. An active agent according to claim 17 or 18, wherein said pathological condition is pulmonary emphysema, adult respiratory difficulty syndrome, interstitial fibrosis, granulomatose sickness, tumour of the lung and pleuritis.

20. A therapeutically active agent according to claim 3 for the treatment of pathological conditions related to the immune response of mammals

21. An active agent according to claim 20, wherein said pathological condition is a virus influenza or an allergic response.

22. A therapeutically active agent according to claim 3 for the treatment of pathological conditions related tomotility of cancer cells.

23. An active agent according to claim 22 wherein said compound is selected from the group consisting of
PIC-(L)-Leu-(S)-Cyt-OH
or
2,4-DMB-(L)-Leu-(S)-Cyt-OH

24. An active agent according claim 22 or 23, wherein said pathological condition is a metastization process in patients affected from tumour.

## Patentansprüche

1. Peptidomimetische Verbindungen der Formel
(a) 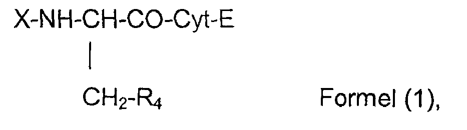
in der
E OH, NH₂, NHOH, N(CH₃)OH oder Ester bedeutet,
R₄ CH-(CH₃)₂, Indol-2-yl, Phenyl, Cyclohexyl oder (CH₂)₃-NH-Fmoc darstellt, Cyt- einen Cyclotryptophanrest bedeutet,
X Xa, 5-Methoxy-1-indanon-3-acetyl, Naphtoyl oder Homoseryl darstellt, wobei Xa einen Rest der Formel 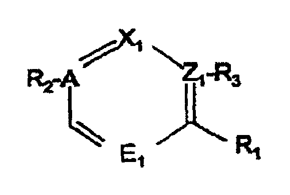 darstellt, in der
X₁: CH, N oder C-OMe bedeutet,
A: C oder N bedeutet,
E₁: CH oder N bedeutet,
Z₁: C oder N bedeutet,
R₁: CO, (CH₂)₂-CO, SO₂, CH₂-CO oder S-CH₂-CO bedeutet,
R₂: OMe, H, NO₂, Cl, OEt oder CH₃ bedeutet,
R₃: OEt, H, OMe bedeutet,
und pharmazeutisch verträgliche Salze, Ester und Amide von allen vorstehend angegebenen Verbindungen.

2. Verfahren zum Bestimmen des therapeutischen Potentials einer peptidomimetischen Verbindung als Inhibitor einer Zink-abhängigen Metall-Proteinase-Aktivität, die mit pathologischen Zuständen von Menschen und Tieren assoziiert ist, umfassend die Schritte:
(a) Durchführen eines ersten Enzym-Hemmtests und Bestimmen eines ersten Niveaus einer Hemmaktivität von peptidomimetischen Verbindungen als Inhibitoren einer Zink-abhängigen Metall-Proteinase, die aus dem Gift von Schlangen extrahiert ist, die zu den Familien Crotalidae und Viperidae gehören;
(b) Screening der Verbindungen auf der Basis des ersten Niveaus der Hemmaktivität durch Ermitteln, ob jede der Verbindungen eine Hemmwirkung auf die Zink-abhängige Metall-Proteinase aus Schlangengift ausübt,
(c) Durchführen eines zweiten Enzym-Hemmtests und Bestimmen eines zweiten Niveaus einer Hemmaktivität der Verbindungen als Inhibitoren einer Zink-abhängigen Metall-Proteinase, die in einem Säuger endogen ist, wobei die Aktivität der Zink-abhängigen Metall-Proteinase mit mindestens einem pathologischen Zustand in dem Säuger assoziiert ist;
(d) Screening der Verbindungen auf der Basis des zweiten Niveaus der Hemmaktivität durch Ermitteln, ob jede der Verbindungen eine Hemmwirkung auf die Zink-abhängige Metall-Proteinase, die in Säugern endogen ist, ausübt; wodurch
Schritt (a) und Schritt (b) eine erste Selektion der getesteten Verbindungen als Inhibitoren der Zink-abhängigen Metall-Proteinase aus Schlangengift und somit als mögliche Inhibitoren von Zink-abhängigen Metall-Proteinasen in Säugern durchführen;
Schritt (c) und Schritt (d) die Fähigkeit der ausgewählten Verbindungen testen, die Zink-abhängigen Metall-Proteinasen, die mit mindestens einem pathologischen Zustand bei Säugern assoziiert sind, zu hemmen,
**dadurch gekennzeichnet, dass** die peptidomimetischen Verbindungen die Formel 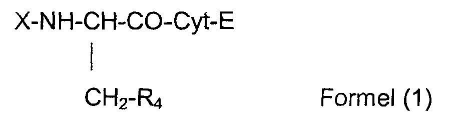 aufweisen, in der X, Cyt, E und R₄ die gleiche Bedeutung gemäß der Definition in Anspruch 1 haben.

3. Therapeutisch wirksames Mittel der Formel 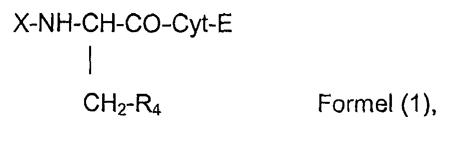 in der X, Cyt, E und R₄ die gleiche Bedeutung gemäß der Definition in Anspruch 1 haben, und pharmazeutisch verträgliche Salze, Ester und Amide davon zum Behandeln von pathologischen Zuständen, die mit der Aktivierung von mindestens einer Zn-Metall-Proteinase aus der Klasse von Zn-abhängigen Metall-Proteinasen, die in Säugergeweben endogen sind, zusammenhängen.

4. Mittel nach Anspruch 3, wobei die Metall-Proteinase, die in Säugern endogen ist, aus Gelatinase oder Collagenase ausgewählt ist.

5. Mittel nach Anspruch 4, wobei die pathologischen Zustände, die mit der Aktivierung von Gelatinase assoziiert sind, Metastasierungsprozesse sind.

6. Mittel nach Anspruch 4, wobei die pathologischen Zustände, die mit der Aktivierung von Collagenase assoziiert sind, Entzündungsreaktionen, rheumatoide Arthritis, Osteoarthritis, Tumorinvasion, Wundheilung, Periodontitis und Hornhautgeschwüre sind.

7. Therapeutisch wirksames Mittel nach einem der Ansprüche 3 bis 6, wobei die Verbindung aus der Gruppe ausgewählt ist, die besteht aus
PIC-(L)-Leu-(S)-Cyt-OH
2,4-DMB-(L)-Leu-(S)-Cyt-OH
5-MIC-(L)-Cha-(S)-Cyt-OH
5-MIC-(L)-Trp-(S)-Cyt-OH
2-QIC-(L)-Leu-(S)-Cyt-OH
6-MNC-(L)-Leu-(S)-Cyt-OH
5-MIA-(L)-Leu-(S)-Cyt-OH
HDC-(L)-Leu-(S)-Cyt-OH
BZS-(L)-Leu-(S)-Cyt-OH
4-MBZ-(L)-Trp-(S)-Cyt-OH
4-CBZ-(L)-Leu-(S)-Cyt-OH

8. Therapeutisch wirksames Mittel nach Anspruch 3 zum Behandeln von pathologischen Zuständen, die mit der Freisetzung des Tumornekrosefaktors durch Zellen von Säugern, einschließlich Menschen, zusammenhängen.

9. Wirksames Mittel nach Anspruch 8, wobei die Verbindung ausgewählt ist aus PIC-(L)-Leu-(S)-Cyt-OH oder 4-MBZ-(L)-Trp-(S)-Cyt-OH.

10. Wirksames Mittel nach Anspruch 8 oder 9, wobei die pathologischen Zustände septischer Schock, multiple Sklerose und Immundefizienz, verursacht durch eine Virusinfektion, sind.

11. Therapeutisch wirksames Mittel nach Anspruch 1 zum Behandeln von pathologischen Zuständen, die mit den toxischen und letalen Effekten von Schlangengift zusammenhängen.

12. Wirksames Mittel nach Anspruch 11, wobei die Verbindung aus der Gruppe ausgewählt ist, die besteht aus
5-MIC-Cha-(S)-Cyt-OH
2-PMTA-(L)-Leu-(S)-Cyt-OH
4-PTA-(L)-Leu-(S)-Cyt-OH
3-APZC-(L)-Leu-(S)-Cyt-OH
7-MBF-(L)-Len-(S)-Cyt-OH
4MQC-(L)-Leu-(S)-Cyt-OH
5-HIC-(L)-Leu-(S)-Cyt-OH
5-MIC-(L)-Phe-(S)-Cyt-OH
PIC-(L)-Leu-(S)-Cyt-OH
(L)-HomoSer-(L)-Leu-(S)-Cyt-OH
5-MIC-(L)-Trp-(S)-Cyt-OH
2-BZF-(L)-Leu-(S)-Cyt-OH
2-QIC-(L)-Leu-(S)-Cyt-OH
5-MIC-(L)-Leu-(S)-OH
2-PZC-(L)-Leu-(S)-Cyt-OH
2-MPA-(L)-Leu-(S)-Cyt-OH
2-EBZ-(L)-Leu-(S)-Cyt-OH
5-MPZ-(L)-Leu-(S)-Cyt-OH
6-MNC-(L)-Leu-(S)-Cyt-OH
5-MIA-(L)-Leu-(S)-Cyt-OH
2,4-DMB-(L)-Leu-(S)-Cyt-OH
4-MBZ-(L)-Trp-(S)-Cyt-OH
4-NBZ-(L)-Leu-(S)-Cyt-OH
4-CBZ-(L)-Leu-(S)-Cyt-OH
3-NIC-(L)-Leu-(S)-Cyt-OH
4-NIC-(L)-Leu-(S)-Cyt-OH
3,4-DMB-(L)-Leu-(S)-Cyt-OH
HDC-(L)-Leu-(S)-Cyt-OH
BZS-(L)-Leu-(S)-Cyt-OH
4-EBZ-(L)-Leu-(S)-Cyt-OH
1-NAF-(L)-Leu-(S)-Cyt-OH

13. Mittel nach Anspruch 11 oder 12, wobei die pathologischen Zustände durch Schlangengift induzierte Blutungen sind.

14. Mittel nach einem der Ansprüche 11 bis 13, wobei das Gift zu Schlangen der Familien Crotalidae und Viperidae gehört.

15. Therapeutisch wirksames Mittel nach Anspruch 3 zum Behandeln von pathologischen Zuständen, die zu inneren Blutungen des Gefäßgewebes führen.

16. Mittel nach Anspruch 15, wobei die pathologischen Zustände innere Mikroblutungen oder Arteriosklerose und andere pathologische Zustände sind, die von Verletzungen des Gefäßgewebes ausgehen.

17. Therapeutisch wirksames Mittel nach Anspruch 3 zum Behandeln von pathologischen Zuständen, die mit der Motilität der Zellen zusammenhängen, die an der Entzündungsreaktion auf bronchopulmonaler Ebene beteiligt sind.

18. Wirksames Mittel nach Anspruch 17, wobei die Verbindung aus der Gruppe ausgewählt ist, die besteht aus
4-MBZ-(L)-Trp-(S)-Cyt-OH,
HDC-(L)-Leu-(S)-Cyt-OH,
2-PZC-(L)-Leu-(S)-Cyt-OH
oder
2-PZC-(L)-Leu-(S)-Cyt-OH.

19. Wirksames Mittel nach Anspruch 17 oder 18, wobei der pathologische Zustand Lungenemphysem, Schocklunge, interstitielle Fibrose, Granulomatose, Lungentumor und Pleuritis ist.

20. Therapeutisch wirksames Mittel nach Anspruch 3 zum Behandeln von pathologischen Zuständen, die mit der Immunreaktion von Säugern zusammenhängen.

21. Wirksames Mittel nach Anspruch 20, wobei der pathologische Zustand eine Virusinfluenza oder eine allergische Reaktion ist.

22. Therapeutisch wirksames Mittel nach Anspruch 3 zum Behandeln von pathologischen Zuständen, die mit der Motilität von Krebszellen zusammenhängen.

23. Wirksames Mittel nach Anspruch 22, wobei die Verbindung aus der Gruppe ausgewählt ist, die aus
PIC-(L)-Leu-(S)-Cyt-OH
oder
2,4-DMB-(L)-Leu-(S)-Cyt-OH besteht.

24. Wirksames Mittel nach Anspruch 22 oder 23, wobei der pathologische Zustand ein Metastasierungsprozess in Patienten ist, die an einem Tumor leiden.

## Revendications

1. Composés peptidomimétiques de formule
(a) 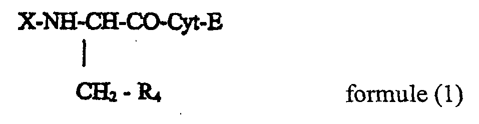
dans laquelle
E indique OH, NH₂, NHOH, N(CH₃)OH ou des esters;
R₄ est CH-(CH₃)₂, indol-2-yle, phényle, cyclohexyle ou (CH₂)₃-NH-Fmoc;
Cyt- indique un résidu de cyclotryptophane,
X est Xa, 5-méthoxy-1-indanone-3-acétyle, naphtoyle ou homoséryle, où Xa est 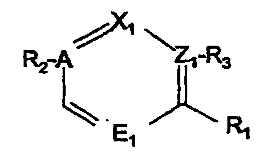
où
X₁ est: CH, N ou C-OMe
A est: C ou N
E₁ est: CH ou N
Z₁ est: C ou N
R₁ est: CO, (CH₂)₂-CO, SO₂, CH₂-CO ou S-CH₂-CO
R₂ est: OMe, H, NO₂, Cl, OEt ou CH₃
R₃ est: OEt, H, OMe
et les sels, esters et amides pharmaceutiquement acceptables de tous les composés figurant ci-dessus.

2. Procédé de détermination du potentiel thérapeutique d'un composé peptidomimétique en tant qu'inhibiteur de l'activité de métalloprotéase dépendante du zinc associée à des états pathologiques de l'homme et de l'animal, comprenant les étapes suivantes :
(a) on effectue un premier essai d'inhibition d'enzyme et on détermine un premier niveau d'activité inhibitrice de composés peptidomimétiques en tant qu'inhibiteurs d'une métalloprotéase dépendante du zinc extraite du venin de serpents appartenant aux familles des Crotalidés et des Vipéridés;
(b) on crible lesdits composés sur la base dudit premier niveau d'activité inhibitrice en évaluant si chacun desdits composés a une activité inhibitrice sur ladite métalloprotéase dépendante du zinc de venin de serpent,
(c) on effectue un deuxième essai d'inhibition d'enzyme et on détermine un deuxième niveau d'activité inhibitrice desdits composés en tant qu'inhibiteurs d'une métalloprotéase dépendante du zinc endogène chez un mammifère, l'activité de ladite métalloprotéase dépendante du zinc étant associée à au moins un état pathologique chez ledit mammifère;
(d) on crible lesdits composés sur la base dudit deuxième niveau d'activité inhibitrice en évaluant si chacun desdits composés a une activité inhibitrice sur ladite métalloprotéase dépendante du zinc endogène chez les mammifères; grâce à quoi
l'étape (a) et l'étape (b) opèrent une première sélection des composés testés en tant qu'inhibiteurs de ladite métalloprotéase dépendante du zinc de venin de serpent et donc en tant qu'inhibiteurs potentiels de métalloprotéases dépendantes du zinc chez des mammifères;
l'étape (c) et l'étape (d) testent la capacité desdits composés sélectionnés à inhiber les métalloprotéases dépendantes du zinc associées à au moins un état pathologique chez des mammifères,
**caractérisé en ce que** lesdits composés peptidomimétiques ont la formule 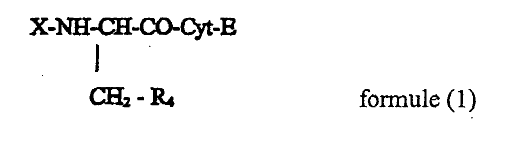 dans laquelle X, Cyt, E et R₄ ont la même signification que dans la revendication 1.

3. Agent thérapeutiquement actif de formule 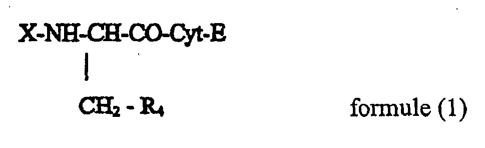 dans laquelle X, Cyt, E et R₄ ont la même signification que dans la revendication 1, et leurs sels, esters et amides pharmaceutiquement acceptables, destinés au traitement d'états pathologiques associés à l'activation d'au moins une métalloprotéase à Zn de la classe des métalloprotéases dépendantes du Zn endogènes dans des tissus de mammifères.

4. Agent selon la revendication 3, ladite métalloprotéase endogène chez les mammifères étant choisie parmi une gélatinase ou une collagénase.

5. Agent selon la revendication 4, lesdits états pathologiques associés à une activation de la gélatinase étant des processus de formation de métastases.

6. Agent selon la revendication 4, lesdits états pathologiques associés à une activation de la collagénase étant des réactions inflammatoires, la polyarthrite rhumatoïde, l'arthrose, l'invasion tumorale, la cicatrisation, la parodontite et les ulcérations cornéennes.

7. Agent thérapeutiquement actif selon l'une quelconque des revendications 3 à 6, dans lequel ledit composé est choisi dans le groupe constitué de
PIC-(L)-Leu-(S)-Cyt-OH
2,4-DMB-(L)-Leu-(S)-Cyt-OH
5-MIC-(L)-Cha-(S)-Cyt-OH
5-MIC-(L)-Trp-(S)-Cyt-OH
2-QIC-(L)-Leu-(S)-Cyt-OH
6-MNC-(L)-Leu-(S)-Cyt-OH
5-MIA-(L)-Leu-(S)-Cyt-OH
HDC-(L)-Leu-(S)-Cyt-OH
BZS-(L)-Leu-(S)-Cyt-OH
4-MBZ-(L)-Trp-(S)-Cyt-OH
4-CBZ-(L)-Leu-(S)-Cyt-OH

8. Agent thérapeutiquement actif selon la revendication 3 destiné au traitement d'états pathologiques associés à la libération du facteur de nécrose tumorale par les cellules de mammifères, y compris de l'homme.

9. Agent actif selon la revendication 8, dans lequel ledit composé est choisi parmi PIC-(L)-Leu-(S)-Cyt-OH ou 4-MBZ-(L)-Trp-(S)-Cyt-OH.

10. Agent actif selon la revendication 8 ou 9, lesdits états pathologiques étant le choc septique, la sclérose en plaques et l'immunodéficience due à une infection virale.

11. Agent thérapeutiquement actif selon la revendication 1 destiné au traitement d'états pathologiques associés aux effets toxiques et létaux du venin de serpent.

12. Agent actif selon la revendication 11, dans lequel ledit composé est choisi dans le groupe constitué de
5-MIC-(L)-Cha-(S)-Cyt-OH
2-PMTA-(L)-Leu-(S)-Cyt-OH
4-PTA-(L)-Leu-(S)-Cyt-OH
3-APZC-(L)-Leu-(S)-Cyt-OH
7-MBF-(L)-Leu-(S)-Cyt-OH
4-MQC-(L)-Leu-(S)-Cyt-OH
5-HIC-(L)-Leu-(S)-Cyt-OH
5-MIC-(L)-Phe-(S)-Cyt-OH
PIC-(L)-Leu-(S)-Cyt-OH
(L)-HomoSer-(L)-Leu-(S)-Cyt-OH
5-MIC-(L)-Trp-(S)-Cyt-OH
2-BZF-(L)-Leu-(S)-Cyt-OH
2-QIC-(L)-Leu-(S)-Cyt-OH
5-MIC-(L)-Leu-(S)-Cyt-OH
2-PZC-(L)-Leu-(S)-Cyt-OH
2-MPA-(L)-Leu-(S)-Cyt-OH
2-EBZ-(L)-Leu-(S)-Cyt-OH
5-MPZ-(L)-Leu-(S)-Cyt-OH
6-MNC-(L)-Leu-(S)-Cyt-OH
5-MIA-(L)-Leu-(S)-Cyt-OH
2,4-DMB-(L)-Leu-(S)-Cyt-OH
4-MBZ-(L)-Trp-(S)-Cyt-OH
4-NBZ-(L)-Leu-(S)-Cyt-OH
4-CBZ-(L)-Leu-(S)-Cyt-OH
3-NIC-(L)-Leu-(S)-Cyt-OH
4-NIC-(L)-Leu-(S)-Cyt-OH
3,4-DMB-(L)-Leu-(S)-Cyt-OH
HDC-(L)-Leu-(S)-Cyt-OH
BZS-(L)-Leu-(S)-Cyt-OH
4-EBZ-(L)-Leu-(S)-Cyt-OH
1-NAF-(L)-Leu-(S)-Cyt-OH

13. Agent selon la revendication 11 ou 12, lesdits états pathologiques étant des hémorragies induites par du venin de serpent.

14. Agent selon l'une quelconque des revendications 11 à 13, ledit venin étant du venin de serpents appartenant aux familles des Crotalidés et des Vipéridés.

15. Agent thérapeutiquement actif selon la revendication 3 destiné au traitement d'états pathologiques qui entraînent des hémorragies internes du tissu vasculaire.

16. Agent thérapeutiquement actif selon la revendication 15, lesdits états pathologiques étant des microhémorragies internes, ou l'artériosclérose et d'autres états pathologiques provenant de lésions du tissu vasculaire.

17. Agent thérapeutiquement actif selon la revendication 3, destiné au traitement d'états pathologiques associés à la motilité des cellules impliquées dans la réponse inflammatoire au niveau broncho-pulmonaire.

18. Agent actif selon la revendication 17, dans lequel ledit composé est choisi dans le groupe constitué de
4-MBZ-(L)-Trp-(S)-Cyt-OH
HDC-(L)- Leu-(S)-Cyt-OH
2-PZC-(L)-Leu-(S)-Cyt-OH
et
2-PZC-(L)-Leu-(S)-Cyt-OH

19. Agent actif selon la revendication 17 ou 18, ledit état pathologique étant l'emphysème pulmonaire, le syndrome de détresse respiratoire de l'adulte, la fibrose interstitielle, la granulomatose, le cancer du poumon et la pleurésie.

20. Agent thérapeutiquement actif selon la revendication 3, destiné au traitement d'états pathologiques associées à la réponse immunitaire chez un mammifère.

21. Agent actif selon la revendication 20, ledit état pathologique étant une grippe virale ou une réaction allergique.

22. Agent thérapeutiquement actif selon la revendication 3, destiné au traitement d'états pathologiques associés à la motilité de cellules cancéreuses.

23. Agent actif selon la revendication 22, dans lequel ledit composé est choisi dans le groupe constitué de
PIC-(L)-Leu-(S)-Cyt-OH
et
2,4-DMB-(L)-Leu-(S)-Cyt-OH

24. Agent actif selon la revendication 22 ou 23, ledit état pathologique étant un processus de formation de métastases chez des patients atteints d'une tumeur.
